# EUROPEAN PATENT APPLICATION

(11) **EP 3 650 046 A1**
(43) Date of publication of application: **13.05.2020**
(21) Application number: 18205183.9
(22) Date of filing: 08.11.2018
(51) Int. Cl.: A61K 47/64, A61K 47/68, A61K 35/28, A61K 9/51, A61P 35/00, A61K 49/00, A61K 38/40, A61K 38/42, A61K 31/7048

(54) **MESENCHYMAL STEM CELL BASED TARGETED ACTIVE INGREDIENT DELIVERY SYSTEM**

(71) Applicant: Cellis AG, 8004 Zurich (CH)
(72) Inventor: TULODZIECKA, Karolina, 02-441 Warszawa (PL); SKORZYNSKI, Marcin, 96-300 Zyrardow (PL); RYGIEL, Tomasz, 01-355 Warsaw (PL); KROL, Magdalena, 02-594 Warszawa (PL)
(74) Representative: Zwicker, Jörk

(57) **Abstract**

The present invention relates to an isolated cellular targeted delivery system comprising a mesenchymal stem cell comprising within said cell a complex of one or more iron-binding proteins and therapeutic or diagnostic agent as well as methods for producing such isolated cellular targeted delivery system and uses of such system for therapy or diagnosis, in particular of cancer.

## Description

The present invention relates to an isolated cellular targeted delivery system comprising a mesenchymal stem cell comprising within said cell a complex of one or more iron-binding proteins and therapeutic or diagnostic agent as well as methods for producing such isolated cellular targeted delivery system and uses of such system for therapy or diagnosis, in particular of cancer.

### BACKGROUND OF THE INVENTION

Mesenchymal stem cells (MSCs) are progenitor cells of non-hematopoietic origin. They are isolated from different types of tissues including adipose tissue, bone marrow, placenta and umbilical cord blood or are derived from Induced Pluripotent Stem Cells (iPSCs). MSCs are defined analytically based on positive (CD73, CD90, and CD105) and negative (CD45, CD34, CD14/CD11b, CD19/CD20/CD79α, and HLA-DR) cell surface markers and functionally based on plastic adherence, the ability to differentiate into osteoblasts, adipocytes, and chondrocytes. However, this definition leaves room for significant phenotypic diversity, and these minimal criteria define a heterogeneous population of cells with implications for clinical development (Phinney DG. (2012) J Cell Biochem 113:2806-2812). Despite of that, MSCs have numerous properties that make them a promising vectors for the delivery of therapeutic agents to tumors and metastatic niches. Firstly the tropism to damaged tissues and tumor sites. It is thought that this migration of MSCs toward the tumor is determined by inflammatory signaling similar to a chronic non-healing wound (Dvorak H. F. (1986) N. Engl. J. Med. 315 1650-1659). It has been shown that MSCs are actively attracted to several cancer like glioma (Smith et al. (2015) Stem Cells Transl. Med. 4 239-251), hepatic carcinoma (Xie et al. (2017) Stem Cells Transl. Med. 6 1120-1131), breast cancer (Ma et al. (2015) Cell Transplant. 24 2585-2599), and also to pre-metastatic niches (Arvelo et al. (2016). Tumour progression and metastasis. Ecancermedicalscience 10:617 10.3332/ecancer.2016.617). Although initial studies were focused on the potential of MSCs to repair damaged tissue via cell replacement, more recently trials are focused on MSCs as a carries of anti-cancer agents due to ability of MSCs for spontaneous endocytosis, and by anchoring of pro-drugs on MSCs surface (Sen Yao et al. (2017) Drug Delivery 24 (1), 1372-1383*;* Denmeade et al. (2003) J Natl Cancer Inst. 95:990-1000; Farinazzo et al. (2018) Sci Rep. May 10;8(1):7473). The present inventors have discovered that MSCs can take up active ingredients complexed with one or more iron-binding proteins and deliver these complexes to or into cells, in particular cancer cells. Based on this observation, the present inventors have developed a targeted delivery system that allows the delivery of therapeutic or diagnostic agents into diseased cells. The advantages are (i) specific delivery, (ii) protection of the therapeutic or diagnostic agents from inactivation in the blood circulation or clearance from the body, (iii) delivery of therapeutic or diagnostic agents to, preferably into cells of poorly or non-vascularized areas of disease, e.g. metastases, hypoxic areas within larger tumours, rheumatic lesions, avascular wounds, skin, (iv) reduced toxicity of therapeutic or diagnostic agents, (v) delivery of therapeutic or diagnostic agents with poor pharmacokinetics, (vi) reduced side effects of the therapeutic or diagnostic agents due to their targeted delivery, (vii) higher treatment efficacy and more sensitive diagnosis with lower doses of the therapeutic or diagnostic agents due to targeted delivery; and/or (viii) lower risk of local tissue injury at the site of therapeutic or diagnostic agents administration due to administration of the therapeutic or diagnostic agents linked with iron-binding protein, which is loaded inside the MSCs.

### SUMMARY OF THE INVENTION

In a first aspect the present invention relates to an isolated targeted delivery system comprising a mesenchymal stem cell (MSC) comprising within said cell a complex of one or more iron-binding proteins and a therapeutic or a diagnostic agent.

In a second aspect the present invention relates to a method of preparation of the isolated targeted delivery system of the first aspect of the invention comprising the following steps:
a) providing purified iron-binding protein;
b) covalently or non-covalently linking a therapeutic or diagnostic agent to an iron-binding protein and/or encapsulating a therapeutic or diagnostic agent in an iron-binding protein;
c) providing a mesenchymal stem cell (MSC); and
d) incubation of the MSC in the presence of the iron-binding protein produced in step b) until the MSC is at least partially loaded with the iron-binding protein produced in step b).

In a third aspect the present invention relates to the isolated targeted delivery system of the first aspect for use as a medicament or a diagnostic.

In a fourth aspect the present invention relates to a pharmaceutical or diagnostic composition comprising the isolated targeted delivery system of the first aspect and a pharmaceutically acceptable carrier and/or suitable excipient(s).

In a fifth aspect the present invention relates to an isolated targeted delivery system of the first aspect of the present invention for use in diagnosing, preventing or treating tumours.

### DETAILED DESCRIPTION OF PREFERRED EMBODIMENTS

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

### DEFINITIONS

To practice the present invention, unless otherwise indicated, conventional methods of chemistry, biochemistry, and recombinant DNA techniques are employed which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. As used in this specification and the appended claims, the singular forms "a", "an", and "the" include plural referents, unless the content clearly dictates otherwise.

The term "targeted drug delivery" refers to the delivery of an therapeutic or diagnostic agent (herein together referred to also as "active ingredient") to a subject, e.g. patient, which results in an increased concentration of the active ingredient in a particular region of the body when compared to other regions of the body of that patient. Preferably, the relative concentrations are compared between a diseased region(s) of the body and other regions of the body having similar access to the blood circulation. In preferred embodiments the concentration of the active ingredient in a given number of cells or a given biopsy volume from the diseased region is at least 10% higher, if compared to the identical number of cells or biopsy volume from a non-diseased region after administration of the targeted delivery system of the present invention, preferably after 2-24 hrs. More preferably, the concentration of the active ingredient in the diseased region of the body of a patient is at least 20%, at least 30%, at least 40%, at least 50%, at least 60%, at least 70% at least 80%, at least 90%, at least 100%, at least 150%, at least 200%, at least 250%, at least 300%, at least 350%, at least 400%, at least 450%, at least 500%, more preferably at least 1000% higher than in a non-diseased region of the body after administration of the targeted delivery system of the present invention, preferably after 2-24 hrs. When assessed on the basis of total body distribution it is preferred that at least 5% of the active ingredient administered to a patient is delivered to the diseased region of the body, preferably at least 10%, more preferably at least 15%. The targeted delivery of the active ingredient limits the potential deleterious effects of an active ingredient to the diseased region of the body.

The term "targeted delivery system" refers to a system that is capable of delivering an active ingredient to the targeted region, i.e. of capable of targeted delivery, preferably within the body of a patient.

The term "active ingredient" means a therapeutically or diagnostically active ingredients and refers to a therapeutic or diagnostic agent.

The terms "therapeutic agent" or "drug" are used synonymously in the context of the present invention and refer to any compound that modifies or modulates cell activity or is capable of being activated, i.e. a prodrug, to modify or modulate cell activity, preferably in the body of a patient. Examples of such active ingredients include so called "small molecules" and peptides. The term "small molecule" is used in the context of the present invention to refer to a hydrocarbon with a molecular mass of below 1.500 g/mol or to pharmaceutically active radioactive isotopes. Preferred, drugs that can be used comprise anti-cancer drugs, pharmaceutically active radioactive isotopes or ferrihydrite.

The term "prodrug" as used in the context of the present invention refers to any active ingredient that, after administration, is metabolized or otherwise converted to a biologically active or more active ingredient (or drug) with respect to at least one property. In comparison to the drug, a prodrug is modified chemically in a manner that makes it, relative to the drug, less active or inactive, but the chemical modification is such that the corresponding drug is generated by metabolic or other biological processes after the prodrug is administered to the patient. A prodrug may for example have, relative to the active drug, altered metabolic stability or transport characteristics, fewer side effects or lower toxicity, or improved flavor (for example, see the reference Nogrady, 1985, Medicinal Chemistry A Biochemical Approach, Oxford University Press, New York, pages 388-392, incorporated herein by reference). A prodrug may be synthesized using reactants other than the corresponding drug.

The terms "diagnostic agent" and "label" are used interchangeably herein and refer to any kind of compound being suitable for diagnostic purposes. Preferred compounds are selected from a fluorescent dye, a radioisotope and a contrast agent. A contrast agent is a dye or other substance that helps to show abnormal areas inside the body. In one embodiment the term label refers to a compound that comprises a chelating agent which forms a complex with divalent or trivalent metal cations. Preferred radioisotopes/fluorescence emitting isotopes are selected from the group consisting of alpha radiation emitting isotopes, gamma radiation emitting isotopes, Auger electron emitting isotopes, X-ray emitting isotopes, fluorescent isotopes, such as ⁶⁵Tb, fluorescence emitting isotopes, such as ¹⁸F, ⁵¹Cr, ⁶⁷Ga, ⁶⁸Ga, ¹¹¹In, ^{99m}Tc, ¹⁴⁰La, ¹⁷⁵Yb, ¹⁵³Sm, ¹⁶⁶Ho, ⁸⁸Y, ⁸⁹Zr, ⁹⁰Y, ¹⁴⁹Pm, ¹⁷⁷Lu, ⁴⁷Sc, ¹⁴²Pr, ¹⁵⁹Gd, ²¹²Bi, ⁷²As, ⁷²Se, ⁹⁷Ru, ¹⁰⁹Pd, ¹⁰⁵Rh, ^{101m15}Rh, ¹¹⁹Sb, ¹²⁸Ba, ¹²³I, ¹²⁴I, ¹³¹I, ¹⁹⁷Hg, ²¹¹At, ¹⁶⁹Eu, ²⁰³Pb, ²¹²Pb, ⁶⁴Cu, ⁶⁷Cu, ¹⁸⁸Re, ¹⁸⁶Re, ¹⁹⁸Au and ¹⁹⁹Ag as well as conjugates and combinations of above with proteins, peptides, small molecular inhibitors, antibodies or other compounds, e.g. ¹⁸F fluorodeoxyglucose (¹⁸F-FDG) or ⁶⁴Cu-porfirin. Preferred fluorescent dyes are selected from the following classes of dyes: Xanthens (e.g. Fluorescein), Acridines (e.g. Acridine Yellow), Oxazines (e.g. Oxazine 1), Cynines (e.g. Cy7 / Cy 3), Styryl dyes (e.g. Dye-28), Coumarines (e.g. Alexa Fluor 350), Porphines (e.g. Chlorophyll B), Metal-Ligand-Complexes (e.g. PtOEPK), Fluorescent proteins (e.g APC, R-Phycoerythrin), Nanocrystals (e.g QuantumDot 705), Perylenes (e.g. Lumogen Red F300) and Phtalocyanines (e.g. IRDYE™700DX) as well as conjugates and combinations of these classes of dyes or fluorescent ⁶⁵Tb emitting. Preferred contrast agents are selected from paramagnetic agents, e.g. Gd, Eu, W and Mn, preferably complexed with a chelating agent. Further options are superparamagnetic iron (Fe) complexes and particles, compounds containing atoms of high atomic number, i.e. iodine for computer tomography (CT), microbubbles and carriers such as liposomes that contain these contrast agents.

The terms "polynucleotide" and "nucleic acid" are used interchangeably herein and are understood as a polymeric or oligomeric macromolecule made from nucleotide monomers. Nucleotide monomers are composed of a nucleobase, a five-carbon sugar (such as but not limited to ribose or 2'-deoxyribose), and one to three phosphate groups. Typically, a polynucleotide is formed through phosphodiester bonds between the individual nucleotide monomers. In the context of the present invention referred to nucleic acid molecules include but are not limited to ribonucleic acid (RNA), deoxyribonucleic acid (DNA), and mixtures thereof such as e.g. RNA-DNA hybrids. The nucleic acids, can e.g. be synthesized chemically, e.g. in accordance with the phosphotriester method (see, for example, Uhlmann, E. & Peyman, A. (1990) Chemical Reviews, 90, 543-584). "Aptamers" are nucleic acids which bind with high affinity to a polypeptide. Aptamers can be isolated by selection methods such as SELEmir146-a (see e.g. Jayasena (1999) Clin. Chem., 45, 1628-50; Klug and Famulok (1994) M. Mol. Biol. Rep., 20, 97-107; US 5,582,981) from a large pool of different single-stranded RNA molecules. Aptamers can also be synthesized and selected in their mirror-image form, for example as the L-ribonucleotide (Nolte et al. (1996) Nat. Biotechnol., 14, 1116-9; Klussmann et al. (1996) Nat. Biotechnol., 14, 1112-5). Forms which have been isolated in this way enjoy the advantage that they are not degraded by naturally occurring ribonucleases and, therefore, possess greater stability.

The term "peptide" or "polypeptide" is used interchangeably in the context of the present invention to refer to a chain of at least two amino acids linked by peptide bonds. Thus, the term "peptide" in the context of the present invention is also used to refer to amino acid chains with more than 50, more than 100 or more than 150 amino acids.

The term "antibody" as used in the context of the present invention refers to a glycoprotein belonging to the immunoglobulin superfamily; the terms antibody and immunoglobulin are often used interchangeably. An antibody refers to a protein molecule produced by plasma cells and is used by the immune system to identify and neutralize foreign objects such as bacteria and viruses. The antibody recognizes a unique part of the foreign target, its antigen.

The term "antibody fragment" as used herein, refers to one or more fragments of an antibody that retain the ability to specifically bind to an antigen. Examples of binding fragments encompassed within the term "antibody fragment" include a fragment antigen binding (Fab) fragment, a Fab' fragment, a F(ab')₂ fragment, a heavy chain antibody, a single-domain antibody (sdAb), a single-chain fragment variable (scFv), a fragment variable (Fv), a V_{H} domain, a V_{L} domain, a single domain antibody, a nanobody, an IgNAR (immunoglobulin new antigen receptor), a di-scFv, a bispecific T-cell engager (BITEs), a dual affinity re-targeting (DART) molecule, a triple body, a diabody, a single-chain diabody, an alternative scaffold protein, and a fusion protein thereof.

The term "diabody" as used within this specification refers to a fusion protein or a bivalent antibody which can bind different antigens. A diabody is composed of two single protein chains which comprise fragments of an antibody, namely variable fragments. Diabodies comprise a heavy chain variable domain (V_{H}) connected to a light-chain variable domain (V_{L}) on the same polypeptide chain (V_{H}-V_{L}, or V_{L}-V_{H}). By using a short peptide connecting the two variable domains, the domains are forced to pair with the complementary domain of another chain and thus, create two antigen-binding sites. Diabodies can target the same (monospecific) or different antigens (bispecific).

A "single domain antibody", refers to antibody fragments consisting of a single, monomeric variable domain of an antibody. Simply, they only comprise the monomeric heavy chain variable regions of heavy chain antibodies produced by camelids or cartilaginous fish. Due to their different origins they are also referred to VHH or VNAR (variable new antigen receptor)-fragments. Alternatively, single-domain antibodies can be obtained by monomerization of variable domains of conventional mouse or human antibodies by the use of genetic engineering. They show a molecular mass of approximately 12-15 kDa and thus, are the smallest antibody fragments capable of antigen recognition. Further examples include nanobodies or nanoantibodies.

The term "antibody mimetic" as used within the context of the present specification refers to compounds which can specifically bind antigens, similar to an antibody, but are not structurally related to antibodies. Usually, antibody mimetics are artificial peptides or proteins with a molar mass of about 3 to 20 kDa which comprise one, two or more exposed domains specifically binding to an antigen. Examples include inter alia the LACI-D1 (lipoprotein-associated coagulation inhibitor); affilins, e.g. human-γ B crystalline or human ubiquitin; cystatin; Sac7D from *Sulfolobus acidocaldarius*; lipocalin and anticalins derived from lipocalins; DARPins (designed ankyrin repeat domains); SH3 domain of Fyn; Kunits domain of protease inhibitors; monobodies, e.g. the 10^{th} type III domain of fibronectin; adnectins: knottins (cysteine knot miniproteins); atrimers; evibodies, e.g. CTLA4-based binders, affibodies, e.g. three-helix bundle from Z-domain of protein A from *Staphylococcus aureus*; Trans-bodies, e.g. human transferrin; tetranectins, e.g. monomeric or trimeric human C-type lectin domain; microbodies, e.g. trypsin-inhibitor-II; affilins; armadillo repeat proteins. Nucleic acids and small molecules are sometimes considered antibody mimetics as well (aptamers), but not artificial antibodies, antibody fragments and fusion proteins composed from these. Common advantages over antibodies are better solubility, tissue penetration, stability towards heat and enzymes, and comparatively low production costs.

The term "sequence identity" is used throughout the specification with regard to polypeptide and nucleotide sequence comparisons. In case where two sequences are compared and the reference sequence is not specified in comparison to which the sequence identity percentage is to be calculated, the sequence identity is to be calculated with reference to the longer of the two sequences to be compared, if not specifically indicated otherwise. If the reference sequence is indicated, the sequence identity is determined on the basis of the full length of the reference sequence indicated by SEQ ID, if not specifically indicated otherwise. For example, a polypeptide sequence consisting of 200 amino acids compared to a reference 300 amino acid long polypeptide sequence may exhibit a maximum percentage of sequence identity of 66.6% (200/300) while a sequence with a length of 150 amino acids may exhibit a maximum percentage of sequence identity of 50% (150/300). If 15 out of those 150 amino acids are different from the respective amino acids of the 300 amino acid long reference sequence, the level of sequence identity decreases to 45%. The similarity of nucleotide and amino acid sequences, i.e. the percentage of sequence identity, can be determined via sequence alignments. Such alignments can be carried out with several art-known algorithms, preferably with the mathematical algorithm of Karlin and Altschul (Karlin & Altschul (1993) Proc. Natl. Acad. Sci. USA 90: 5873-5877), with hmmalign (HMMER package, http://hmmer.wustl.edu/) or with the CLUSTAL algorithm (Thompson, J. D., Higgins, D. G. & Gibson, T. J. (1994) Nucleic Acids Res. 22, 4673-80) available e.g. on http://www.ebi.ac.uk/Tools/clustalw/ or on http://www.ebi.ac.uk/Tools/clustalw2/index.html or on http://npsa-pbil.ibcp.fr/cgi-bin/npsa_automat.pl?page=/NPSA/npsa_clustalw.html. Preferred parameters used are the default parameters as they are set on http://www.ebi.ac.uk/Tools/clustalw/ or http://www.ebi.ac.uk/Tools/clustalw2/index.html. The grade of sequence identity (sequence matching) may be calculated using e.g. BLAST, BLAT or BlastZ (or BlastX). BLAST protein searches are performed with the BLASTP program, score = 50, word length = 3. To obtain gapped alignments for comparative purposes, Gapped BLAST is utilized as described in Altschul et al. (1997) Nucleic Acids Res. 25: 3389-3402. When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1:154-162) or Markov random fields. Structure based alignments for multiple protein sequences and/or structures using information from sequence database searches, available homologs with 3D structures and user-defined constraints may also be used (Pei J, Grishin NV: PROMALS: towards accurate multiple sequence alignments of distantly related proteins. Bioinformatics 2007, 23:802-808; 3DCoffee@igs: a web server for combining sequences and structures into a multiple sequence alignment. Poirot O, Suhre K, Abergel C, O'Toole E, Notredame C. Nucleic Acids Res. 2004 Jul 1;32:W37-40.). When percentages of sequence identity are referred to in the present application, these percentages are calculated in relation to the full length of the longer sequence, if not specifically indicated otherwise.

The term "mesenchymal stem cell" or "MSC" is used in the context of the present invention to refer to adult stem cells which are non-haematopoietic, multipotent stem cells with the capacity to differentiate into mesodermal lineage such as osteocytes, adipocytes and chondrocytes as well ectodermal (neurocytes) and endodermal lineages (hepatocytes). MSCs express cell surface markers like cluster of differentiation (CD)73, CD90, and CD105 and lack the expression of CD45, CD34, CD14/CD11b, CD19/CD20/CD79a, and HLA (human leucocyte antigen)-DR. Human MSCs for the first time were reported in the bone marrow and till now they have been isolated from various tissues, including adipose tissue, placenta, amniotic fluid, endometrium, dental tissues, umbilical cord blood and umbilical cord tissue (Wharton's jelly). They also have been derived (i.e. differentiated) from Induced Pluripotent Stem Cells (iPSCs). Thus, in a preferred embodiment of the invention, the MSC is selected from the group consisting of an umbilical cord MSC, a bone marrow MSC, an adipose MSC, a placenta MSC, a dental MSC, an amniotic fluid MSC, an endometrium MSC, and an iPSC-derived MSC. Preferably, it is an umbilical cord blood MSC or an umbilical cord tissue (or Wharton's jelly) MSC. Furthermore, it is preferred that the MSC is a human MSC. The present inventors have observed that such MSCs stem cells can be loaded with the complex according to the invention and deliver it into cancer cells.

"Expressing" means in this respect that the majority of cells within a population of cells or essentially all cells express the marker (also called surface antigen herein). In this context and also with reference to other cellular surface antigens, the term "expresses" indicates that the surface antigen is produced within the cell and detectably exposed on the surface of a cell. The level of expression and, thus the number of surface antigens detectably exposed on the surface of a cell can vary greatly among different cells. Generally, a cell is considered to be positive, i.e. is indicated to be "⁺", for a cellular surface antigen, if at least 5, preferably at least 10 copies of the surface antigen are detectably exposed on the surface of the cell. The skilled person is well aware of how to detect, quantify and select for cells, which are positive (or negative) for a given cellular surface antigen. Preferred methods include Fluorescence Activated Cell Sorting (FACS). In this technology fluorescently labelled antibodies are used to bind to cellular surface antigens of a population of cells, the cells are subsequently isolated into single cells and based on fluorescence intensity measured for the single cell, characterized as being positive or negative for the given cellular surface antigen. It is well known in the art how to quantify the number of proteins expressed or produced in a cell using flow cytometry and Becton Dickinson Quantibrite™ bead method (see e.g. Pannu, K.K., 2001, Cytometry. 2001 Dec 1;45(4):250-8) or mass spectrometry (see, e.g. Milo, R., 2013, Bioessays, 35(12): 1050-1055).

In a first aspect the present invention relates to an isolated targeted delivery system comprising a mesenchymal stem cell (MSC), comprising within said cell a complex of one or more iron-binding proteins and a therapeutic or a diagnostic agent.

The ability of a given MSC or of a population thereof to internalize iron-binding proteins depends on the expression of receptors involved in this internalization process. Receptors that lead to internalization of ferritin comprise, e.g. TfR, CXCR4, CD163, and TIM-2. The skilled person is well aware how to measure the amount of uptake of an iron-binding protein and preferred method of measuring the uptake are described in the Example Section below.

The phrase "complex of one or more iron-binding proteins and an active ingredient" as used in the context of the present invention refers to a composition in which one or more molecules of the active ingredient are covalently or non-covalently linked to one or more iron-binding proteins. The covalent or non-covalent linkage between the one or more iron-binding proteins and the one more active ingredient can be direct or indirect. In the latter case the active ingredient is linked to the iron-binding protein via a linker or spacer. Linker or spacers are known to the skilled artisan, such as polyalanine, polyglycin, carbohydrates, (CH₂)ₙ groups or polypeptide linkers. The skilled artisan will, thus, be able to select the respective suitable linker(s) or spacer(s) depending on the respective application. If the iron-binding proteins form cages like, e.g. ferritin, than the term "complex" also encompasses the enclosure of active ingredients within the cage even in the absence of a covalent or non-covalent bond between the protein(s) and the active compound(s). The formation of the complex allows the transport of the active ingredient into the cell when the cell is internalizing the iron-binding protein. Thus, it is preferred that the active ingredients are linked to the iron-binding protein in a way that does not interfere with the transport mechanism. This can be easily tested by the skilled person using uptake assays known in the art and described in the Example Section below. It is preferred that the complex is sufficiently stable to survive the transport within the cell to the target region within the body. Thus, it is preferred that the complex rather than the active ingredient alone is delivered to the cells or into the cells in the target region. This property also reduces possible deleterious effects, e.g. cytotoxicity, of the active ingredient to the MSC. If active ingredients are covalently linked to the iron-binding proteins such linkage is preferably through amino acids residues known to be located in surface areas that are not involved in binding to the cellular receptors required for cellular uptake of the iron-binding proteins. Iron-binding proteins used in the context of the present invention can form stable non-covalently linked complexes with a wide variety of active ingredients.

MSCs originating from a subject to be diagnosed or treated and loaded with the complex can be autologous to the subject. It is also envisioned that subjects are MHC typed prior to diagnosis or treatment with the targeted delivery of the present invention and that the cell used for a given subject is MHC matched to the subject. The cell is preferably obtained from the subject or a healthy donor.

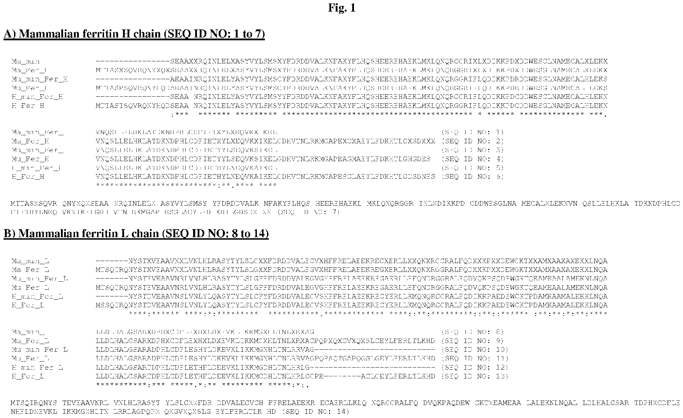

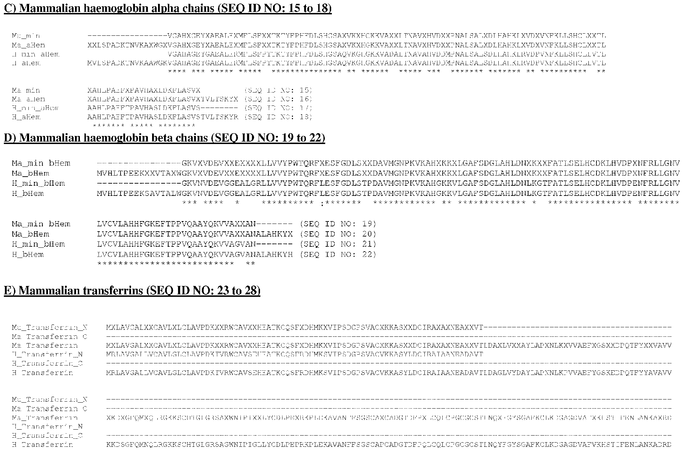

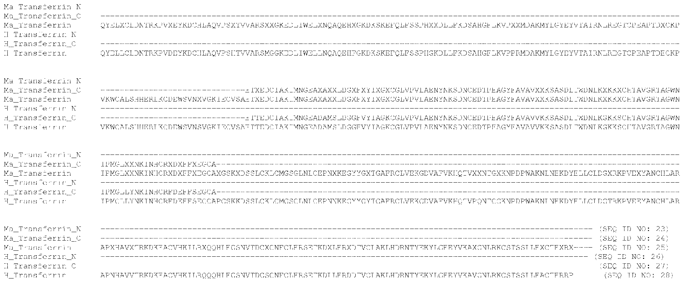

In a preferred embodiment of the targeted delivery system of the present invention the iron-binding protein is selected from the group consisting of ferritin, preferably heavy (H) type ferritin, light (L) ferritin and/or mitochondrial ferritin; haemoglobin, preferably haemoglobin A, haemoglobin AS, haemoglobin SC, haemoglobin C, haemoglobin D, haemoglobin E, haemoglobin F, haemoglobin H; haemoglobin-haptoglobin complex, haemopexin, transferrin; and lactoferrin. The terms ferritin; haemoglobin, preferably haemoglobin A, haemoglobin AS, haemoglobin SC, haemoglobin C, haemoglobin D, haemoglobin E, haemoglobin F, haemoglobin H; haemoglobin-haptoglobin complex, hemopexin, transferrin; and lactoferrin encompass structural variants of the naturally occurring proteins and, thus relates to proteins that have at least 70%, preferably at least 75%, more preferably at least 80%, more preferably at least 85%, more preferably at least 90%, more preferably at least 95% more preferably at least 100% of the ability of the respective wild-type protein to bind iron ion(s). The iron-binding proteins used in the context of the present invention are preferably of mammalian, more preferably mouse, rat, dog, ape, in particular, chimpanzee, or human, most preferably of human origin. Consensus sequences of the preferred iron-binding proteins used in the context of the present invention are shown in Fig. 1 below. Preferred structural variants are based on the sequences indicated in Fig. 1. The residues marked with X vary among different mammalian ferritins, transferrins, and haemoglobins. The alteration of these residues is not crucial for the ability of the proteins to bind to iron ions. Accordingly, it is preferred that amino acid mutations or deletions effect one or more of the residues marked with an X.

Plasma proteins have always been privileged carriers for the delivery of active pharma ingredients in cancer therapy. Thus, albumin, the most abundant plasma protein, is currently used in therapeutic protocols for the delivery of taxane molecules and doxorubicin derivatives (Larsen MT et al. 2016, Mol Cell Ther 27;4:3).

Human transferrin and ferritin proteins have been considered as effective carriers for the delivery of small molecules or toxin-conjugates to specifically target cancer cells. To date, in spite of considerable efforts, no successful transferrin or ferritin drug complexes have however reached the clinic (Luck AN et al. 2013, Adv Drug Deliv Rev 65(8):1012-9).

Ferritin has a cage architecture and capability of iron-binding which could be used to encapsulate drugs inside its cavity. Ferritins are not abundant in plasma, but can be readily produced in high yield as recombinant proteins in common protein expression vectors such as Escherichia coli cells. Ferritins H- or L- chains are encoded as small protein monomer (21 kDa and 19 KDa for H and L chains, respectively) capable of a 24-mer assembly into a cage-like structure, delimiting a 8 nm diameter cavity. The present inventors noted in the context of working on the present invention that H-ferritin homopolymers, represent a preferred protein construct in order to specifically deliver encapsulated drugs to MSCs expressing TfR. Furthermore H-ferritin targets complex active ingredients to the cell nucleus (and therefore directly delivers DNA-binding proteins into the nucleus).

Purified transferrin can be efficiently conjugated to some anticancer drugs through covalent linkers that are appropriately released inside the cells (Beyer U et al. 1998, J Med Chem 41(15):2701-2708). In case of transferrin, only lysine groups on the protein surface are ready available for covalent attachment.

Haemoglobin has been considered in the past as a possible drug carrier, due to its versatility in chemical conjugation with drugs, its abundance and relative stability in the blood (Somatogen, 1993, WO1993008842 A1). Nevertheless, the lack of receptor targeting properties did not foster biomedical applications other than blood substitutes or antisickling agent. The MSCs described in this application moved haemoglobin center stage as a target specific drug carrier. Haemoglobin can be readily covalently linked to appropriate drug conjugates, host hydrophobic drug molecules within the heme binding pocket or even transport small cytotoxic molecules linked to the heme iron. Hb can be easily modified by selective attachment of the appropriate drug conjugate to the beta93 cysteine residue, the only titratable cystein on the protein surface. Maleimido functionalized drugs, such as the tubuline inhibitor MonomethylAuristatin (MMAE) or the DNA crosslinking drug Pyrrolobenzodiazepine dimer (PBD) are most notable examples of extremely potent cytotoxics that can be readily and specifically attached to the relevant cys beta93 residue.

Alternatively, lysine residues on the Hb surface (at least 10 titratable lysine residues per Hb tetramer) may be easily amenable to drug conjugation through cleavable succinimide linkers. Haemoglobin also offers a unique capability of releasing non covalently bound heme group at acidic pH values. Apo-haemoglobin thus obtained is capable of hosting several hydrophobic molecules within the empty heme pocket, as shown in the case of paclitaxel (Meng Z et al. 2015 J Pharm Sci 104(3):1045-55).

Whatever the conjugation/adsorption/binding method, haemoglobin (Hb), transferrin (Tf) and ferritins were shown by the present inventions to be privileged drug carriers, once loaded into appropriate cell systems with tumour targeting properties, e.g. activated macrophages. The easy, fast, cheap and safe purification procedure of these protein also provide a tremendous added value.

Based on sequences of mammalian H-type ferritins, L-type ferritins, haemoglobin alpha chains, haemoglobin beta chains and transferrins a consensus sequence was determined for each of these proteins. These are shown in Fig. 1 in SEQ ID NO: 2, 7, 9, 14, 16, 20, and 25, respectively. On this basis but also on the basis of deletion and structural analysis disclosed in the prior art a minimal fragment was determined for H-type ferritins, L-type ferritins, haemoglobin alpha chains, and haemoglobin beta chains sufficient for uptake by MSCs. These are shown in SEQ ID NO: 1, 3, 5 (H-type ferritin); 8, 10 12 (L-type ferritin), 15 and 17 (haemoglobin alpha chain) and 19 and 21 (haemoglobin beta chain. Transferrin comprises a N-terminally located domain and a C-terminally located domain that are necessary for binding iron and uptake by MSCs, if comprised in one polypeptide and positioned between 100 to 450 amino acids apart, preferably between 150 to 400, more preferably between 200 to 350 amino acids and more preferably 250 to 320 amino acids apart. The N-terminal domain comprises amino acids 1 to 82 of full length consensus transferrin (SEQ ID NO: 25) or full length human transferrin (SEQ ID NO: 28). The C-terminal domain comprises amino acids 396 to 510 of full length consensus transferrin (SEQ ID NO: 25) or full length human transferrin (SEQ ID NO: 28). In each case an X is indicated in the consensus sequence it independently stands for any amino acid and characterizes an amino acid not or only poorly conserved among mammalian H-type ferritins, which can be mutated without or little detriment to the iron-binding properties of the respective iron-binding protein. It is preferred that X in each case takes on the meaning of the amino acid of the respective human iron-binding protein aligning with X. This information can be taken, e.g. from Fig. 1, which shows alignments of the consensus sequences with human an in some instances mouse proteins.

Preferred H-type ferritins comprise or consist of the amino acid sequence indicated in SEQ ID NO: 1 and variants thereof having at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a H-type ferritin consisting of the amino acid sequence according to SEQ ID NO: 1 to be taken up by MSCs. Within SEQ ID NO: 1 X at position 5 may be present or absent, if present it means any amino acid, preferably Ile, X at position 6 means any amino acid, preferably Asn, X at position 14 means any amino acid, preferably Tyr, X at position 24 means any amino acid, preferably Tyr or Cys, X at position 66 means any amino acid, preferably Phe, X at position 68 means any amino acid, preferably Gln, X at position 75 means any amino acid, preferably Arg or Cys, X at position 90 means any amino acid, preferably His, X at position 94 means any amino acid, preferably Ser or Asn, X at position 120 may be present or absent, if present it means any amino acid, preferably His or Tyr, more preferably His, X at position 123 means any amino acid, preferably Asn or Ser, more preferably Asn, X at position 128 means any amino acid, preferably Ala or Ser, more prefrably Ala.

In a preferred embodiment the H-type ferritin comprises or consists of murine ferritin according to SEQ ID NO: 3. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a H-type ferritin consisting of the amino acid sequence according to SEQ ID NO: 3 to be taken up by MSCs.

In a preferred embodiment the H-type ferritin comprises or consists of human ferritin according to SEQ ID NO: 5. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a H-type ferritin consisting of the amino acid sequence according to SEQ ID NO: 5 to be taken up by MSCs.

In a preferred embodiment the H-type ferritin comprises or consists of a mammalian consensus sequence derived from aligning full length H-type ferritins according to SEQ ID NO: 2 or 7, with 2 being preferred. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a H-type ferritin consisting of the amino acid sequence according to SEQ ID NO: 2 or 7, with 2 being preferred to be taken up by MSCs. In SEQ ID NO: 2 X at position 6 can be any naturally occurring amino acid, preferably Pro, X at position 14 can be any naturally occurring amino acid, preferably His, X at position 16 can be any naturally occurring amino acid, preferably Asp, X at position 21 may be present or absent, if present it means any amino acid, preferably Ile, X at position 22 means any amino acid, preferably Asn, X at position 30 can be any naturally occurring amino acid, preferably Tyr, X at position 40 can be any naturally occurring amino acid, preferably Tyr or Cys, more preferably Tyr, X at position 82 can be any naturally occurring amino acid, preferably Phe, X at position 84 can be any naturally occurring amino acid, preferably Gln, X at position 91 can be any naturally occurring amino acid, preferably Arg or Cys, more preferably Cys, X at position 106 can be any naturally occurring amino acid, preferably His, X at position 110 can be any naturally occurring amino acid, preferably Asn or Ser, more preferably Asn, X at position 137 can be any naturally occurring amino acid, preferably His or Tyr, more preferably His, X at position 140 can be any naturally occurring amino acid, preferably Asn or Ser, more preferably Asn, X at position 145 can be any naturally occurring amino acid, preferably Ala or Ser, more preferably Ala, X at position 164 can be any naturally occurring amino acid, preferably Ala or Ser, more preferably Ser, X at position 166 can be any naturally occurring amino acid, preferably Met or Leu, preferably Leu, X at position 178 can be any naturally occurring amino acid, preferably Asp or His, more preferably Asp, X at position 181 is absent or any naturally occurring amino acid, preferably Asn, X at position 182 is absent or any naturally occurring amino acid, preferably Glu, X at position 183 is absent or any naturally occurring amino acid, preferably Ser. In SEQ ID NO: 7 X at position 6 can be any naturally occurring amino acid, preferably Pro X at position 14 can be any naturally occurring amino acid, preferably His, X at position 16 can be any naturally occurring amino acid, preferably Asp, X at position 21 may be present or absent, if present it means any amino acid, preferably Ile, X at position 29 can be any naturally occurring amino acid, preferably Tyr, X at position 81 can be any naturally occurring amino acid, preferably Phe, X at position 83 can be any naturally occurring amino acid, preferably Gln, X at position 105 can be any naturally occurring amino acid, preferably His, X at position 144 can be any naturally occurring amino acid, preferably Ala or Ser, more preferably Ala, X at position 180 is absent or any naturally occurring amino acid, preferably Asn, X at position 181 is absent or any naturally occurring amino acid, preferably Glu, X at position 182 is absent or any naturally occurring amino acid, preferably Ser.

In a preferred embodiment the H-type ferritin comprises or consists of murine full length ferritin according to SEQ ID NO: 4 being preferred. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a murine H-type ferritin consisting of the amino acid sequence according to SEQ ID NO: 4 to be taken up by MSCs.

In a preferred embodiment the H-type ferritin comprises or consists of human full length ferritin according to SEQ ID NO: 6 being preferred. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a human H-type ferritin consisting of the amino acid sequence according to SEQ ID NO: 6 to be taken up by MSCs.

Preferred L-type ferritins comprise or consist of the amino acid sequence indicated in SEQ ID NO: 8 and variants thereof having at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a L-type ferritin consisting of the amino acid sequence according to SEQ ID NO: 8 to be taken up by MSCs. In SEQ ID NO: 8 X at position at position 5 can be any naturally occurring amino acid, preferably Asp or Glu, more preferably Asp, X at position 12 can be any naturally occurring amino acid, preferably Arg or Ser, more preferably Ser, X at position 17 can be any naturally occurring amino acid, preferably Ser or Arg, more preferably Ser, X at position 19 can be any naturally occurring amino acid, preferably Arg or Gln, more preferably Gln, X at position 29 can be any naturally occurring amino acid, preferably Phe, X at position 30 can be any naturally occurring amino acid, preferably Tyr or Phe, more preferably Tyr, X at position 42 can be any naturally occurring amino acid, preferably Ser or Gly, more preferably Ser, X at position 56 can be any naturally occurirng amino acid, preferably Ala or Tyr, more preferably Tyr, X at position 61 can be any naturally occurring amino acid, preferably Glu or Lys, more preferably Lys, X at position 62 can be any naturally occurring amino acid, preferably Met or Phe, more preferably Met, X at position 65 can be any naturally occurring amino acid, preferably Asp or Gln, more preferably Gln, X at position 75 can be any naturally occurring amino acid, preferably Ile or Val, more preferably Ile, X at position 76 can be any naturally occurring amino acid, preferably Lys or Gln, more preferably Lys, X at position 79 can be any naturally occurring amino acid, preferably Ala or Ser, more preferably Ala, X at position 80 can be any naturally occurring amino acid, preferably Glu or Gln, more preferably Gln, X at position 87 can be any naturally occurring amino acid, preferably Pro or Gln, more preferably Pro, X at position 88 can be any naturally occurring amino acid, preferably Glu or Asp, more preferably Asp, X at position 91 can be any naturally occurring amino acid, preferably Glu or Lys, more preferably Lys, X at position 94 can be any naturally occurring amino acid, preferably Met or Leu, more preferably Leu, X at position 96 can be any naturally occurring amino acid, preferably Met or Leu, more preferably Met, X at position 99 can be any naturally occurring amino acid, preferably Lys or Asn, preferably Lys, X at position 115 can be any naturally occurring amino acid, preferably Thr or Ala, more preferably Thr, X at position 119 can be any naturally occurring amino acid, preferably Leu, X at position 125 can be any naturally occurring amino acid, preferably Ser or Thr, more preferably Thr, X at position 127 can be any naturally occurring amino acid, preferably Tyr or Phe, more preferably Phe, X at position 130 can be any naturally occurring amino acid, preferably Lys or Glu, more preferably Glu, X at position 140 can be any naturally occurring amino acid, preferably Asp or Asn, more preferably Asp, X at position 146 can be any naturally occurring amino acid, preferably Arg or His, more preferably His, and X at position 148 can be any naturally occurring amino acid, preferably Leu or Val, more preferably Leu.

In a preferred embodiment the L-type ferritin comprises or consists of murine L-type ferritin according to SEQ ID NO: 10. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a L-type ferritin consisting of the amino acid sequence according to SEQ ID NO: 10 to be taken up by MSCs.

In a preferred embodiment the L-type ferritin comprises or consists of human ferritin according to SEQ ID NO: 12. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a L-type ferritin consisting of the amino acid sequence according to SEQ ID NO: 12 to be taken up by MSCs.

In a preferred embodiment the L-type ferritin comprises or consists of a mammalian consensus sequence derived from aligning full length H-type ferritins according to SEQ ID NO: 9 or 14, with 9 being preferred. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a L-type ferritin consisting of the amino acid sequence according to SEQ ID NO: 9 or 14, with 9 being preferred to be taken up by MSCs. In SEQ ID NO: 9 X at position 12 X at position 12 can be any naturally occurring amino acid, preferably Asp or Glu, more preferably Asp, X at position 19 can be any naturally occurring amino acid, preferably Ser or Arg, more preferably Ser, X at position 24 can be any naturally occurring amino acid, preferably Ser or Arg, more preferably Ser, X at position 26 can be any naturally occurring amino acid, preferably Arg or Gln, more preferably Gln, X at position 36 can be any naturally occurring amino acid, preferably Phe, X at position 37 can be any naturally occurring amino acid, preferably Tyr or Phe, more preferably Tyr, X at position 47 can be any naturally occurring amino acid, preferably Ser or Gly, more preferably Ser, X at position 63 can be any naturally occurring amino acid, preferably Ala or Tyr, more preferably Tyr, X at position 68 can be any naturally occurring amino acid, preferably Glu or Lys, more preferably Lys, X at position 69 can be any naturally occurring amino acid, preferably Met or Phe, more preferably Met, X at position 72 can be any naturally occurring amino acid, preferably Asp or Gln, more preferably Gln, X at position 82 can be any naturally occurring amino acid, preferably Ile or Val, more preferably Ile, X at position 83 can be any naturally occurring amino acid, preferably Lys or Gln, more preferably Lys, X at position 86 can be any naturally occurring amino acid, preferably Ala or Ser, more preferably Ala, X at position 87 can be any naturally occurring amino acid, preferably Glu or Gln, more preferably Gln, X at position 94 can be any naturally occurring amino acid, preferably Pro or Gln, more preferably Pro, X at position 95 can be any naturally occurring amino acid, preferably Glu or Asp, more preferably Asp, X at position 98 can be any naturally occurring amino acid, preferably Glu or Lys, more preferably Lys, X at position 101 can be any naturally occurring amino acid, preferably Met or Leu, more preferably Leu, X at position 103 can be any naturally occurring amino acid, preferably Met or Leu, more preferably Met, X at position 106 can be any naturally occurring amino acid, preferably Lys or Asn, preferably Lys, X can be any naturally occurring amino acid, X at position 126 can be any naturally occurring amino acid, preferably Leu, X at position 132 can be any naturally occurring amino acid, preferably Ser or Thr, more preferably Thr, X at position 134 can be any naturally occurring amino acid, preferably Tyr or Phe, more preferably Phe, X at position 137 can be any naturally occurring amino acid, preferably Lys or Glu, more preferably Glu, X at position 147 can be any naturally occurring amino acid, preferably Asp or Asn, more preferably Asp, X at position 153 can be any naturally occurring amino acid, preferably Arg or His, more preferably His, X at position 155 can be any naturally occurring amino acid, preferably Leu or Val, more preferably Leu, X at position 161 can be absent or any naturally occurring amino acid, preferably Ala, X at position 163 can be absent or any naturally occurring amino acid, preferably Thr, X at position 166 can be absent or any naturally occurring amino acid, preferably Pro, andX at position 168 can be any naturally occurring amino acid, preferably Gly or Ala, more preferably Ala. In SEQ ID NO: 14 X at position 36 can be any naturally occuring amino acid, preferably Phe, X at position 37 can be any naturally occurring amino acid, preferably Tyr or Phe, more preferably Tyr, X at position 94 can be any naturally occurring amino acid, preferably Pro or Gln, more preferably Pro, X at position 126 can be any naturally occurring amino acid, preferably Leu, X at position 137 can be any naturally occurring amino acid, preferably Lys or Glu, more preferably Glu, X at position 147 can be any naturally occurring amino acid, preferably Asp or Asn, more preferably Asp, X can be any naturally occurring amino acid, X at position 163 can be absent or any naturally occurring amino acid, preferably Thr, X at position 166 can be absent or any naturally occurring amino acid, preferably Pro, X at position 168 can be any naturally occurring amino acid, preferably Gly or Ala, more preferably Ala.

In a preferred embodiment the L-type ferritin comprises or consists of murine full length ferritin according to SEQ ID NO: 11 being preferred. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a murine L-type ferritin consisting of the amino acid sequence according to SEQ ID NO: 11 to be taken up by MSCs.

In a preferred embodiment the L-type ferritin comprises or consists of human full length ferritin according to SEQ ID NO: 13 being preferred. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a human L-type ferritin consisting of the amino acid sequence according to SEQ ID NO: 13 to be taken up by MSCs.

In a preferred embodiment the alpha haemoglobin comprises or consists of a minimal mammalian consensus sequence derived from aligning full length alpha haemoglobins according to SEQ ID NO: 15 Preferred comprise or consist of the amino acid sequence indicated in SEQ ID NO: 15 and variants thereof having at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of an alpha haemoglobin consisting of the amino acid sequence according to SEQ ID NO: 15 to be taken up by MSCs.

In a preferred embodiment the alpha haemoglobin comprises or consists of a minimal human amino acid sequence derived of human alpha haemoglobin according to SEQ ID NO: 17. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of an alpha haemoglobin consisting of the amino acid sequence according to SEQ ID NO: 17 to be taken up by MSCs.

In a preferred embodiment the alpha haemoglobin comprises or consists of a mammalian consensus sequence derived from aligning full length alpha haemoglobins according to SEQ ID NO: 16. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of an alpha haemoglobins consisting of the amino acid sequence according to SEQ ID NO: 16 to be taken up by MSCs.

In a preferred embodiment the alpha haemoglobin comprises or consists of human full length alpha haemoglobin according to SEQ ID NO: 18. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a human full length alpha haemoglobin consisting of the amino acid sequence according to SEQ ID NO: 18 to be taken up by MSCs.

In a preferred embodiment the alpha haemoglobin comprises or consists of a minimal mammalian consensus sequence derived from aligning full length beta haemoglobins according to SEQ ID NO: 19 and variants thereof having at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a beta haemoglobin consisting of the amino acid sequence according to SEQ ID NO: 19 to be taken up by MSCs.

In a preferred embodiment the alpha haemoglobin comprises or consists of a minimal human amino acid sequence derived of human beta haemoglobin according to SEQ ID NO: 21. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a beta haemoglobin consisting of the amino acid sequence according to SEQ ID NO: 21 to be taken up by MSCs.

In a preferred embodiment the beta haemoglobin comprises or consists of a mammalian consensus sequence derived from aligning full length beta haemoglobins according to SEQ ID NO: 20. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of an beta haemoglobins consisting of the amino acid sequence according to SEQ ID NO: 20 to be taken up by MSCs.

In a preferred embodiment the beta haemoglobin comprises or consists of human full length beta haemoglobin according to SEQ ID NO: 22. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a human full length beta haemoglobin consisting of the amino acid sequence according to SEQ ID NO: 22 to be taken up by MSCs.

In a preferred embodiment the transferrin comprises or consists of a mammalian consensus sequence derived from aligning full length alpha haemoglobins according to SEQ ID NO: 25. Thus, particularly, preferred transferrins comprise or consist of the amino acid sequence indicated in SEQ ID NO: 25 and of variants thereof having at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a transferrin consisting of the amino acid sequence according to SEQ ID NO: 25 to be taken up by MSCs.

In a preferred embodiment the transferrin comprises or consists of human transferrin according to SEQ ID NO: 28. Accordingly, preferred structural variants have at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a transferrin consisting of the amino acid sequence according to SEQ ID NO: 28 to be taken up by MSCs.

The iron-binding properties of transferrins are dependent on a N-terminally and C-terminally located domain. Thus, in a preferred embodiment the transferrin used in the present invention comprises at least the N-terminal domain according to SEQ ID NO: 23 and the C-terminal domain according to SEQ ID NO: 24. Preferred transferrin comprise proteins that comprise the amino acid sequence indicated in SEQ ID NO: 23 and 24 as well as variants thereof having at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a transferrin consisting of the amino acid sequence according to SEQ ID NO: 23 and 24 to be taken up by MSCs. SEQ ID NO: 23 or 24 indicates a consensus sequence of mammalian transferrins.

Thus, in a preferred embodiment the transferrin used in the present invention comprises at least the N-terminal domain according to SEQ ID NO: 26 and the C-terminal domain according to SEQ ID NO: 27. Preferred transferrin comprise proteins that comprise the amino acid sequence indicated in SEQ ID NO: 26 and 27 as well as variants thereof having at least 70% amino acid identity, more preferably at least 75% amino acid identity, more preferably at least 80% amino acid identity, more preferably at least 85% amino acid identity, more preferably at least 90% amino acid identity, more preferably at least 95% amino acid identity and in each case at least 70% of the ability of a transferrin consisting of the amino acid sequence according to SEQ ID NO: 26 and 27, respectively, to be taken up by MSCs.

Preferred ferritins, also comprise proteins that, irrespective of the given amino acid sequence, conform to the 24-mer subunit assembly of a four helix bundle protein module, falling within given sequence alignments of distantly related proteins as defined by 3D structure based alignments.

It is a surprising observation of the present inventors that MSCs can take up complexes comprising one or more ferritin and one or more active ingredient. In a preferred embodiment of the targeted delivery system of the present invention the therapeutic agent is selected from the group consisting of a protein, a nucleic acid, a chemical non-protein non-nucleic acid compound with a molecular weight of less than 1.5 kD, more preferably less than 1 kD, preferably an immunomodulatory drug or an anticancer drug, in particular a cytostatic drug, cytotoxic drug and prodrugs thereof; an anti-arteriosclerotic drug; and anti-inflammatory drug; and photosensitizing compound; a virus, in particular oncolytic virus; and a α or ß radiation emitting radioisotope, which also emit a cell damaging amount of γ radiation, preferably selected from the group consisting of lutetium-177, ytterbium-90, iodine-131, samarium-153, phosphorus-32, caesium-131, palladium-103, radium-233, iodine-125, and boron-10 or a cell damaging amount of α radiation, preferably selected from the group consisting of actinium-225, bismuth-213, lead-212, and polonium-212.

Preferred anticancer drugs are selected from an apoptosis/autophagy or necrosis-inducing drug. An apoptosis/autophagy or necrosis-inducing drug can be any drug that is able to induce apoptosis/autophagy or necrosis effectively even in cells having an abnormality in cell proliferation. These drugs are preferably used in complexes with one or more ferritins.

Preferred anticancer drugs are selected from the group consisting of an apoptosis-inducing drug, an alkylating substance, anti-metabolites, antibiotics, epothilones, nuclear receptor agonists and antagonists, an anti-androgene, an anti-estrogen, a platinum compound, a hormone, a antihormone, an interferon, an inhibitor of cell cycle-dependent protein kinases (CDKs), an inhibitor of cyclooxygenases and/or lipoxygenases, a biogeneic fatty acid, a biogenic fatty acid derivative, including prostanoids and leukotrienes, an inhibitor of protein kinases, an inhibitor of protein phosphatases, an inhibitor of lipid kinases, a platinum coordination complex, an ethyleneimine, a methylmelamine, a triazine, a vinca alkaloid, a pyrimidine analog, a purine analog, an alkylsulfonate, a folic acid analog, an anthracendione, a substituted urea, and a methylhydrazin derivative, an ene-diyne antibiotic, a tubulin polymerization inhibitor such as a maytansinoid or an auristatine derivate, immune check-point inhibitor, and an inhibitor of tumour-specific protein or marker, preferably a Rho-GDP-dissociation inhibitor, more preferably Grp94.

Other preferred anticancer drugs are selected from the group consisting of acediasulfone, aclarubicine, ambazone, aminoglutethimide, L-asparaginase, azathioprine, banoxantrone, bendamustine, bleomycin, busulfan, calcium folinate, carboplatin, carpecitabine, carmustine, celecoxib, chlorambucil, cis-platin, cladribine, cyclophosphamide, cytarabine, dacarbazine, dactinomycin dapsone, daunorubicin, dibrompropamidine, diethylstilbestrole, docetaxel, doxorubicin, enediynes, epirubicin, epothilone B, epothilone D, estramucin phosphate, estrogen, ethinylestradiole, etoposide, flavopiridol, floxuridine, fludarabine, fluorouracil, fluoxymesterone, flutamide fosfestrol, furazolidone, gemcitabine, gonadotropin releasing hormone analog, hexamethylmelamine, hydroxycarbamide, hydroxymethylnitrofurantoin, hydroxyprogesteronecaproat, hydroxyurea, idarubicin, idoxuridine, ifosfamide, interferon α, irinotecan, leuprolide, lomustine, lurtotecan, mafenide sulfate olamide, mechlorethamine, medroxyprogesterone acetate, megastrolacetate, melphalan, mepacrine, mercaptopurine, methotrexate, metronidazole, mitomycin C, mitopodozide, mitotane, mitoxantrone, mithramycin, nalidixic acid, nifuratel, nifuroxazide, nifuralazine, nifurtimox, nimustine, ninorazole, nitrofurantoin, nitrogen mustards, oleomucin, oxolinic acid, pentamidine, pentostatin, phenazopyridine, phthalylsulfathiazole, pipobroman, prednimustine, prednisone, preussin, procarbazine, pyrimethamine, raltitrexed, rapamycin, rofecoxib, rosiglitazone, salazosulfapyridine, scriflavinium chloride, semustine streptozocine, sulfacarbamide, sulfacetamide, sulfachlopyridazine, sulfadiazine, sulfadicramide, sulfadimethoxine, sulfaethidole, sulfafurazole, sulfaguanidine, sulfaguanole, sulfamethizole, sulfamethoxazole, co-trimoxazole, sulfamethoxydiazine, sulfamethoxypyridazine, sulfamoxole, sulfanilamide, sulfaperin, sulfaphenazole, sulfathiazole, sulfisomidine, staurosporin, tamoxifen, taxol, teniposide, tertiposide, testolactone, testosteronpropionate, thioguanine, thiotepa, tinidazole, topotecan, triaziquone, treosulfan, trimethoprim, trofosfamide, UCN-01, vinblastine, vincristine, vindesine, vinblastine, vinorelbine, and zorubicin, preferably selected from the group consisting of auristatin, banoxantrone, bendamustine, chlorambucil, chaliceamycin, cyclophosphamide dynemycin A, maytansine, melphalan, mertansine, and neocazinostatin, most preferably banoxantrone, bendamustine, chlorambucil, cyclophosphamide, pyrrolobenzodiazepine and melphalan.

Immunomodulatory drugs are drugs that promote or inhibit the activity of immune cells. They can be natural or synthetic agonists or antagonists of Pattern Recognition Receptors, particularly Toll-like Receptors, NOD-like receptors (NLR) or RIG-I-like receptors (RLR). Physiologically, these receptors recognize class of signals known as pathogen-associated molecular patterns (PAMPs) and/or damage-associated molecular patterns (DAMPs).

It is particularly preferred that the anticancer drug is a proliferation inhibiting protein, preferably a cell cycle inhibitor or an antibody or antibody mimetic that specifically binds to a target on or within a cell in the targeted tissue that modulates the disease status of the cell, preferably a proliferation promoting protein, or a nucleic acid, preferably encoding a proliferation inhibiting protein or an antibody or antibody mimetic that specifically binds to a target on or within a cell in the targeted tissue that modulates the disease status of the cell, preferably a proliferation promoting protein or a siRNA or DNAzyme.

Preferred examples of antibodies to be used in the context of the present invention are single chain antibodies, antibody fragments, nanobodies, light or heavy chains, variable light or variable heavy chains, or diabodies. Preferred antibody fragments comprise a fragment antigen binding (Fab) fragment, a Fab' fragment, a F(ab')2 fragment, a heavy chain antibody, a single-domain antibody (sdAb), a single-chain fragment variable (scFv), a fragment variable (Fv), a VH domain, a VL domain, a single domain antibody, a nanobody, an IgNAR (immunoglobulin new antigen receptor), a di-scFv, a bispecific T-cell engager (BITEs), a dual affinity re-targeting (DART) molecule, a triple body, a diabody, a single-chain diabody, and a fusion protein thereof.

If the therapeutic agent is a nucleic acid it is preferred that it is a miRNA, siRNA, DNAzyme or a nucleic acid encoding a pharmaceutically active protein, e.g. an antibody, an antibody mimetic, a cytokine, a prodrug-converting enzyme or the like.

As has been outlined above, the targeted delivery system of the present invention can deliver active ingredients to hypoxic areas. The use of active ingredients which are activated under hypoxic conditions adds a further specificity to the targeting and/or further reduces adverse effects of the active ingredients. Thus, in particularly preferred embodiments the active ingredient is a hypoxia-activated prodrug. The backbone of all the hypoxia-activated prodrugs is the presence of one of five different chemical moieties (nitro groups, quinines, aromatic and aliphatic N-oxides and transition metals) that are enzymatically reduced under hypoxic conditions in tissue. Hypoxia-activated prodrugs are any prodrug that is less active or inactive, relative to the corresponding drug, and comprises the drug and one or more bioreducible groups. Such hypoxia-activated prodrugs include all prodrugs activated by a variety of reducing agents and reducing enzymes, including without limitation single electron transferring enzymes (such as cytochrome P450 reductases) and two electron transferring (or hydride transferring) enzymes. According to preferred embodiment of the invention hypoxia-activated prodrug is TH-302. Methods of synthesizing TH-302 are described in PCT application WO 07/002931 and WO 08/083101. Preferably examples of such prodrugs are selected from the class I group consisting of: benzotriazine N-oxides, apaziquone (EO9), tirapazamine (TPN) and SN30000; or class II group consisting of: nitro compounds PR-104A, TH-302, TH-4000, and AQ4N.

In a preferred embodiment of the targeted delivery system of the present invention diagnostic agent is selected from a fluorescent dye, a fluorescence emitting isotope, a radioisotope, a detectable polypeptide or nucleic acid encoding such a detectable polypeptide and a contrast agent.

In a preferred embodiment of the targeted delivery system of the present invention the diagnostic agent comprises a chelating agent which forms a complex with divalent or trivalent metal cations.

In a preferred embodiment of the targeted delivery system of the present invention the chelating agent is selected from the group consisting of 1,4,7,10-tetraazacyclododecane-N,N',N,*N*'-tetraacetic acid (DOTA), ethylenediaminetetraacetic acid (EDTA), 1,4,7-triazacyclononane-1,4,7-triacetic acid (NOTA), triethylenetetramine (TETA), iminodiacetic acid, Diethylenetriamine-N,N,N',N',N"-pentaacetic acid (DTPA) and 6-Hydrazinopyridine-3-carboxylic acid (HYNIC).

In a preferred embodiment of the targeted delivery system of the present invention the contrast agent comprises a paramagnetic agent, preferably selected from Gd, Eu, W and Mn, or ferrihydride.

In a preferred embodiment of the targeted delivery system of the present invention the radioisotope/fluorescence emitting isotope is selected from the group consisting of alpha radiation emitting isotopes, gamma radiation emitting isotopes, Auger electron emitting isotopes, X-ray emitting isotopes, fluorescence emitting isotopes, such as ¹⁸F, ⁵¹Cr, fluorescent ⁶⁵Tb, ⁶⁷Ga, ⁶⁸Ga, ¹¹¹In, ^{99m}Tc, ¹⁴⁰La, ¹⁷⁵Yb, ¹⁵³Sm, ¹⁶⁶Ho, ⁸⁸Y, ⁹⁰Y, ¹⁴⁹Pm, ¹⁷⁷Lu, ⁴⁷Sc, ¹⁴²Pr, ¹⁵⁹Gd, ²¹²Bi, ⁷²As, ⁷²Se, ⁹⁷Ru, ¹⁰⁹Pd, ¹⁰⁵Rh, ^{101m15}Rh, ¹¹⁹Sb, ¹²⁸Ba, ¹²³I, ¹²⁴I, ¹³¹I, ¹⁹⁷Hg, ²¹¹At, ¹⁶⁹Eu, ²⁰³Pb, ²¹²Pb, ⁶⁴Cu, ⁸⁹Zr, ⁶⁷Cu, ¹⁸⁸Re, ¹⁸⁶Re, ¹⁹⁸Au and ¹⁹⁹Ag as well as conjugates and combinations of above with proteins, peptides, small molecular inhibitors, antibodies or other compounds (e.g. ¹⁸F-FDG or ⁶⁴Cu-porfirin).

In a preferred embodiment of the targeted delivery system of the present invention the fluorescence dye is selected from the group consisting of the following classes of fluorescent dyes: Xanthens, Acridines, Oxazines, Cynines, Styryl dyes, Coumarines, Porphines, Metal-Ligand-Complexes, Fluorescent proteins, Nanocrystals, Perylenes and Phtalocyanines as well as conjugates and combinations of these classes of dyes.

In a preferred embodiment of the targeted delivery system of the present invention the detectable polypeptide is an autofluorescent protein, preferably green fluorescent protein or any structural variant thereof with an altered adsorption and/or emission spectrum.

In a preferred embodiment of the isolated targeted delivery system of the present invention the bond(s) between the iron-binding protein(s) and the active ingredient comprised in the complex are covalent and/or non-covalent; and/or the active ingredient comprised in the complex is entrapped/encapsulated by the iron-binding protein, preferably ferritin or multimers thereof. In one embodiment the covalent and/or non-covalent linking is indirect through a linker or spacer. If the formation of covalent bonds is desired, relevant thiol, amino or carboxyl groups of the iron-binding proteins are used to covalently link active ingredients directly or indirectly to the one or more iron-binding protein. It is also envisioned that different active ingredients are comprised in the complex. For example, one type of active ingredient may be linked to an iron-binding protein (non-covalently linked), while another type is encapsulated. This approach utilizes different release rates of the active ingredients from the iron-binding protein once delivered to the targeted tissue and/or cells. For example, drug derivatives acting as active ingredient can be covalently attached to ferritin molecule either on the surface of the 24-mer or within the internal cavity by exploiting the reactivity of relevant thiol, amino or carboxyl groups. The types of such useful reactions are well known in the art and can be adopted by the person skilled in the art to the concrete active ingredient without any additional work. Examples of such reactions are described in Behrens CR, Liu B. Methods for site-specific drug conjugation to antibodies. MAbs. 2014 Jan-Feb;6(1):46-53.

In a second aspect the present invention relates to a method of preparation of the isolated targeted delivery system of the first aspect comprising steps of
a) providing purified iron-binding protein;
b) covalently or non-covalently linking a therapeutic or diagnostic agent to and/or encapsulating a therapeutic or diagnostic agent in an iron-binding protein;
c) providing a mesenchymal stem cell (MSC); and
d) incubating the MSC in the presence of the iron-binding protein produced in step b) until the MSC is at least partially loaded with the iron-binding protein produced in step b).

The formation of the adduct between the protein and the drug moiety may be a non-covalent drug molecule linking to the target protein and can be described as follows: In the case of ferritin, drugs can be typically encapsulated within the internal cavity (physical confinement) by exploiting the association dissociation properties of the ferritin macromolecule itself. Drug molecules are held in place by non-covalent interactions with aminoacid residues within the cavity internal surface. Haemoglobin macromolecules also offer the possibility of non-covalent linking of selected drug molecules that may be hosted within the heme binding pocket of haemoglobin itself. The heme can be displaced by the pocket and be replaced by drugs with appropriate hydrophobicity profile. In a further aspect, all proteins considered in the present invention may be covalently attached to drug molecules modified by specific active linker moieties reactive towards thiol or amino groups of the protein itself. As such, ferritins or haemoglobin may be linked to cysteine thiol reactive drugs bearing a peptide based cleavable linker (e.g. cathepsin sensitive valine-citrulline sequence and para-aminobenzylcarbamate spacer). As a notable example, the antimitotic agent monomethyl auristatin E (MMAE) has been used. The peptide-based linker linkss the protein to the cytotoxic compound in a stable manner so the drug is not easily released from the protein under physiologic conditions and help prevent toxicity to healthy cells and ensure dosage efficiency. The protein drug adduct thus generated is capable of attaching to the selected receptor types, i.e. CD163 for haemoglobin and TfR for ferritin or transferrin, respectively. Once bound, the protein drug adduct is internalised by endocytosis and thus selectively taken up by targeted cells. The vesicle containing the drug is fused with lysosomes and lysosomal cysteine proteases, particularly cathepsin B start to break down valine-citrulline linker and MMAE is no longer bound to the antibody and is released directly into the tumour environment.

Alternatively, DM1-SMCC is and efficient mertansine derivative bearing a linker that specifically bind to lysine residues generating a covalent complex with ferritin, haemoglobin or transferrin in a reaction that has been successfully described for antibodies. In particular, haemoglobin, ferritin or transferrin can be reacted with DM1-SMCC thus providing a covalent protein-drug adduct that can be cleaved inside cells and releases the active drug in a time-dependent manner. The suppression of microtubule dynamics by DM1 induces mitotic arrest and cell death.

The term "full load" is used in the context of the present invention to refer to the maximum amount of iron-binding protein, preferably ferritin, complexed with an active ingredient that can be taken up by the MSC.

The invention also relates to an isolated targeted delivery system producible with the method of the second aspect.

In a third aspect the present invention relates to the isolated targeted delivery system of the present invention for use as a medicament or a diagnostic.

In a fourth aspect the present invention relates to a pharmaceutical composition comprising the isolated targeted delivery system of the invention and (i) a pharmaceutically acceptable carrier and/or (ii) suitable excipient(s).

Since the isolated targeted delivery system comprises living cells, it is preferred that carriers and excipients are chosen in such to keep the cells alive.

"Pharmaceutically acceptable" means approved by a regulatory agency of the Federal or a state government or listed in the U.S. Pharmacopeia or other generally recognized pharmacopeia for use in animals, and more particularly in humans.

The term "carrier", as used herein, refers to a pharmacologically inactive substance such as but not limited to a diluent, excipient, surfactants, stabilizers, physiological buffer solutions or vehicles with which the therapeutically active ingredient is administered. Such pharmaceutical carriers can be liquid or solid. Liquid carrier include but are not limited to sterile liquids, such as saline solutions in water and oils, including but not limited to those of petroleum, animal, vegetable or synthetic origin, such as peanut oil, soybean oil, mineral oil, sesame oil and the like. Saline solutions and aqueous dextrose and glycerol solutions can also be employed as liquid carriers, particularly for injectable solutions. A saline solution is a preferred carrier when the pharmaceutical composition is administered intravenously. Examples of suitable pharmaceutical carriers are described in "Remington's Pharmaceutical Sciences" by E. W. Martin.

Suitable pharmaceutical "excipients" include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like.

"Surfactants" include anionic, cationic, and non-ionic surfactants such as but not limited to sodium deoxycholate, sodium dodecylsulfate, Triton X-100, and polysorbates such as polysorbate 20, polysorbate 40, polysorbate 60, polysorbate 65 and polysorbate 80.

"Stabilizers" include but are not limited to mannitol, sucrose, trehalose, albumin, as well as protease and/or nuclease antagonists.

"Physiological buffer solution" include but are not limited to sodium chloride solution, demineralized water, as well as suitable organic or inorganic buffer solutions such as but not limited to phosphate buffer, citrate buffer, tris buffer (tris(hydroxymethyl)aminomethane), HEPES buffer ([4 (2 hydroxyethyl)piperazino]ethanesulphonic acid) or MOPS buffer (3 morpholino-1 propanesulphonic acid). The choice of the respective buffer in general depends on the desired buffer molarity. Phosphate buffer are suitable, for example, for injection and infusion solutions.

The term "adjuvant" refers to agents that augment, stimulate, activate, potentiate, or modulate the immune response to the active ingredient of the composition at either the cellular or humoral level, e.g. immunologic adjuvants stimulate the response of the immune system to the actual antigen, but have no immunological effect themselves. Examples of such adjuvants include but are not limited to inorganic adjuvants (e.g. inorganic metal salts such as aluminium phosphate or aluminium hydroxide), organic adjuvants (e.g. saponins or squalene), oil-based adjuvants (e.g. Freund's complete adjuvant and Freund's incomplete adjuvant), cytokines (e.g. IL-1β, IL-2, IL-7, IL-12, IL-18, GM-CFS, and INF-γ) particulate adjuvants (e.g. immuno-stimulatory complexes (ISCOMS), liposomes, or biodegradable microspheres), virosomes, bacterial adjuvants (e.g. monophosphoryl lipid A, or muramyl peptides), synthetic adjuvants (e.g. non-ionic block copolymers, muramyl peptide analogues, or synthetic lipid A), or synthetic polynucleotides adjuvants (e.g polyarginine or polylysine).

In a fifth aspect the present invention relates to the isolated targeted delivery system of the present invention for use in diagnosing, preventing or treating tumours, preferably a solid tumour, preferably breast cancer, pancreatic cancer, bladder cancer, lung cancer, colon cancer, or a tumour having hypoxic areas, inflammatory disease or ischemic areas in skin wounds or after organ infarctus (heart) or ischemic retina.

The term "treatment" as used herein includes all types of preventive and/or therapeutic interventions medically allowed for the purpose of cure, temporary remission, prevention, etc. for different purposes including delaying or stopping the progress of a disease, making a lesion regress or disappear, preventing onset, or inhibiting recurrence.

The targeted delivery system according to the present invention enables tumour delivery of the active ingredients, which normally would not be able to reach the tumour (for example, due to solubility problems). It also enables the delivery of active ingredients to the hypoxic tumours or to the hypoxic areas of the tumour. This system also provides for delivery of an active ingredient to any area within an organism subjected to hypoxic conditions, for example during ischaemic incidents, or undergoing an inflammatory process.

As mentioned above the present invention provides also the method for active ingredient delivery into the tumour mass. This method comprises preparation of MSCs which enables highly efficient iron-binding protein (ferritin, haemoglobin and/or transferring) uptake by the MSCs, wherein said ferritin, haemoglobin and/or transferrin carry an active ingredient (for example a drug/prodrug), tumour targeting and iron-binding protein transfer to the cancer cell, where the active ingredient is released.

The present invention exploits MSCs loaded with iron-binding proteins linked with an active ingredient as a delivery system to target the tumour. Unsatisfactory response of the tumours to chemotherapy or difficulties in their detection using imaging methods are mainly related to an altered penetration of the active ingredients to the hypoxic areas due to poor vasculature. However, these avascular regions attract MSCs to migrate even in areas far away from blood vessels. Therefore, they constitute a delivery system of particles to the tumour mass. A promising example of such particles is iron-binding protein. However, when used as single agents they do not reach hypoxic regions, similarly to other compounds and accumulate in other organs.

The present inventors linked active ingredients to haemoglobin or transferrin using chemical methods and loaded it into MSCs. The inventors observed that loaded MSCs transfer iron-binding protein with encapsulated active ingredients into the cancer cells. This method allows precise administration of the active ingredients to the tumour site, avoiding their accumulation in other organs.

### Brief Description of Drawings

- **Fig. 1:**: Panel (A) shows a minimal active fragment of a consensus amino acid sequence among mammalian ferritin H chains and two full length consensus sequences based on several mammalian ferritin H chains (see SEQ ID NO: 1, 2 and 7, respectively) as well as a minimal and full length amino acid sequence of mouse (SEQ ID NO: 3 and 4) and human (SEQ ID NO: 5 and 6) ferritin H chain. Panel (B) shows a a minimal active fragment of a consensus amino acid sequence among mammalian ferritin L chains and two full length consensus sequences based on several mammalian ferritin L chains (see SEQ ID NO: 8, 9 and 14, respectively) as well as a minimal and full length amino acid sequence of mouse (SEQ ID NO: 10 and 11) and human (SEQ ID NO: 12 and 13) ferritin L chain.. Panel (C) shows a minimal active fragment of a consensus amino acid sequence among mammalian haemoglobin alpha chains and one full length consensus sequences based on several mammalian haemoglobin alpha chain (see SEQ ID NO: 15 and 16, respectively) as well as a minimal and full length amino acid sequence of human (SEQ ID NO: 17 and 18) haemoglobin alpha chain. Panel (D) shows a minimal active fragment of a consensus amino acid sequence among mammalian haemoglobin beta chains and a full length consensus sequences based on several mammalian haemoglobin beta chain (see SEQ ID NO: 19 and 20, respectively) as well as a minimal and full length amino acid sequence of human (SEQ ID NO: 21 and 22) haemoglobin beta chain. Panel (E) shows a N- and C-terminal minimal active fragment of a consensus amino acid sequence among mammalian transferrins (SEQ ID NO: 23 and 24) and a full length consensus sequences based on several mammalian transferrins (SEQ ID NO: 25) as well as a N- and C-terminal minimal active fragment of a human transferrin (SEQ ID NO: 26 and 27) and full length amino acid sequence of human transferrin (SEQ ID NO: 28). In the consensus sequences X indicates a position that is variable and stands for any natural amino acid. Preferably, in each case X in dependently of other X stands for the amino acid present in the human protein.

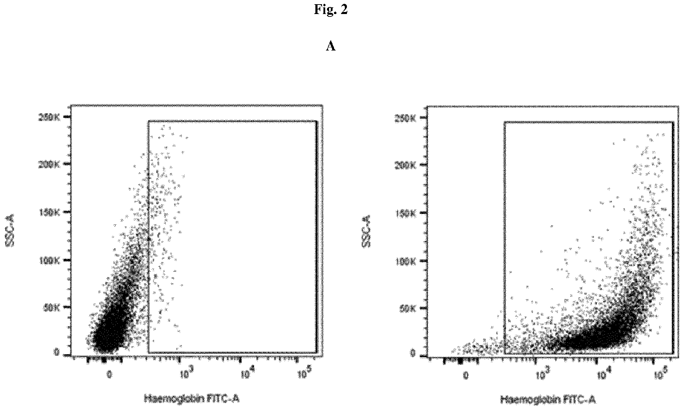

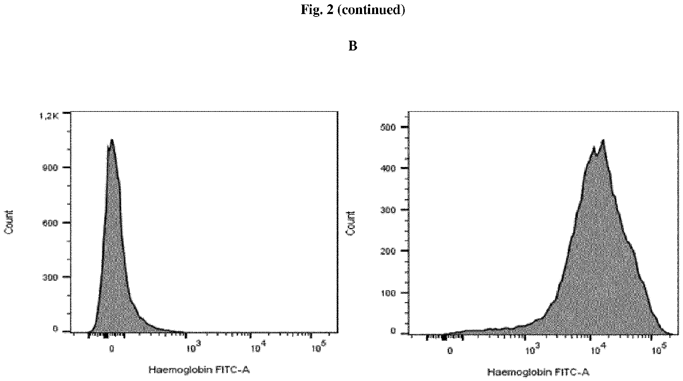

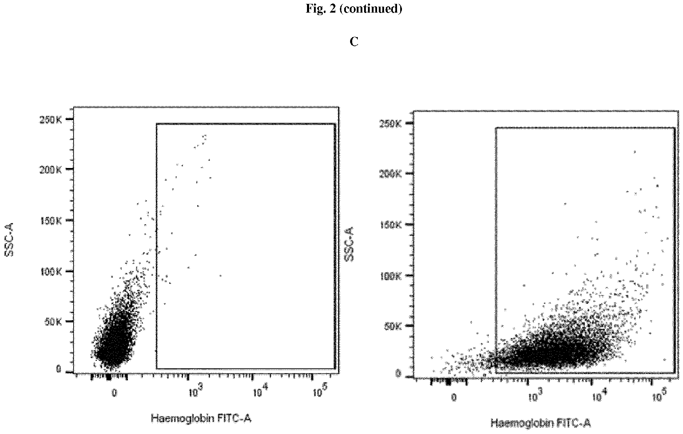

- **Fig. 2:**: **A.** Human umbilical Mesenchymal Stem Cells (MSC) were cultured until passage five. For the experiments cells were loaded with 0.6 mg/ml Haemoglobin-Alexa488 (Ft) with minimal 97% efficacy. Left: Hb⁻, Right: Hb⁺. **B**. The mean fluorescence intensity was 4 times higher in MSC cells loaded with Haemoglobin-Alexa488 than in control group. Left: Hb⁻, Right: Hb⁺. C. MSCs loaded with haemoglobin-Alexa488 were co-cultured for 24 hours with human cancer cell lines HeLa, SCOV3 or MDA-MB-231 in ratio 2:1. Results were analyzed by flow cytometry. Data on graph represent example of results obtained in this experiment. Approximately 96% of HeLa, SCOV3 or MDA-MB-231 were positive for Haemoglobin-Alexa488. Left: MSC co-culture ⁻Hb, Right: MSC co-culture Hb⁺.

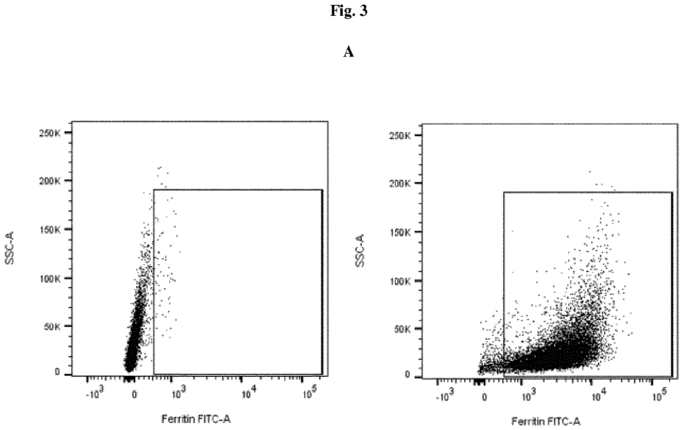

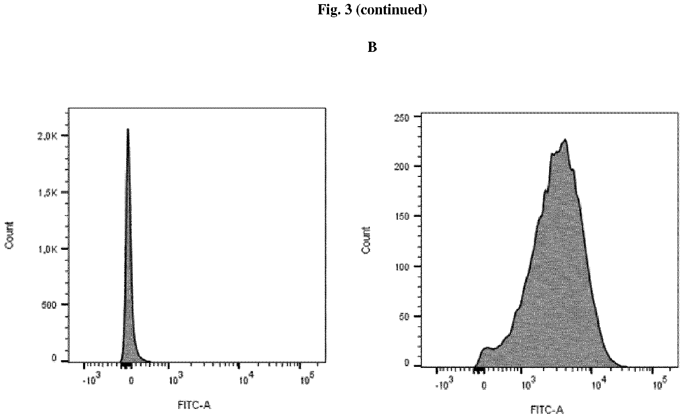

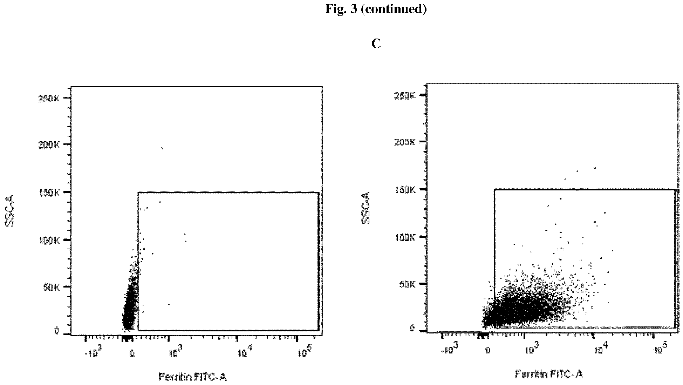

- **Fig. 3:**: **A.** Human umbilical Mesenchymal Stem Cells (MSC) were cultured until passage five. For the experiments cells were loaded with 0.6 mg/ml Ferritin-Alexa488 (Ft) with minimal 95% efficacy. Left: Ft⁻, Right: Ft⁺. **B**. The mean fluorescence intensity was 3 times higher in MSC cells loaded with Ferritin-Alexa488 than in control group. Left: Ft⁻, Right: Ft⁺. C. MSCs loaded with haemoglobin-Alexa488 were co-cultured for 24 hours with human cancer cell lines HeLa, SCOV3 or MDA-MB-231 in ratio 2:1. Results were analysed by flow cytometry. Data on the graph represent example of results obtained in this experiment. Approximately 92% of HeLa, SCOV3 or MDA-MB-231 were positive for Haemoglobin-Alexa488. Left: MSC co-culture Ft⁻, Right: MSC co-culture Ft⁺.

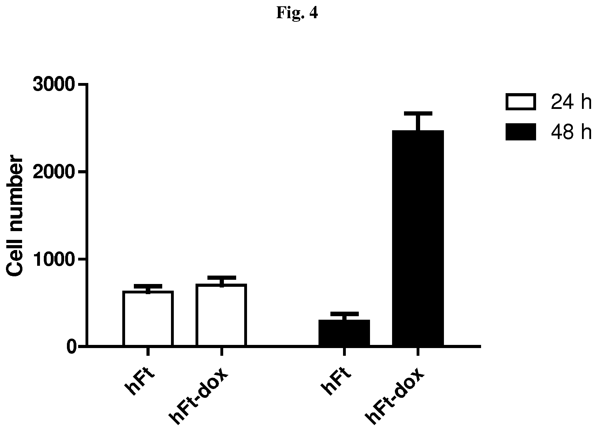

- **Fig. 4:**: Number of dead HeLa cells in co-culture with human ferritin-doxorubicin (hFt-dox)loaded UC-MSC. Human umbilical Mesenchymal Stem Cells (MSC) were cultured until passage five. MSCs loaded with plain ferritin (hFt) and ferritin-doxorubicin (hFt-dox) were co-cultured with human cancer cell lines HeLa, SCOV3 or MDA-MB-231 in ratio 2:1 for 24 and 48 hours. Influence of ferritin doxorubicin influence on cancer cells were analysed by flow cytometry. After 24 hours in co-culture number of dead cells was low both in control hFt and hFt-dox samples, whereas after 48 hours in co-culture number of dead cells increased about 8 times.

### Example Section

### Example 1 - Stem cell loading

Umbilical Cord Mesenchymal Stem cells (UC-MSC) were cultured in DMEM 1000, 1:100 penicillin/streptomycin, 20% FBS, 8 ng/ml bFGF. Media was changed every 2 days. Experiments were carried out on UC-MSCs at passage 5.

Ferritin and haemoglobin complexes were produced as described in WO 2016/207257 (see in particular Examples 5 and 6).

Human UC-MSCs were loaded in suspension with fluorescently labelled haemoglobin-Alexa488, ferritn-Alexa488 or ferritin loaded with anticancer drug doxorubicin (ferritin-doxorubicin) as follows: UC-MSCs were detached from culture dish with 0,25% Trypsin-EDTA then cells were mixed with 0,6 mg/ml of haemoglobin-Alexa488, ferritn-Alexa488 and ferritn-doxorubicin in PBS to final cell density 10⁶ per ml. Next cells were incubated for 1 hour at 37 °C, 5% CO₂ and washed once in PBS afterwards. Cells were immediately used to set up co-culture with cancer cells.

### Example 2 - Delivery to cancer cells

Cancer cell lines HeLa, SKOV3 and MDA-MB-231 were cultures in RPMI, 10%FBS, 1:100 penicillin/streptomycin, 1:100 L-glutamine. Media was changed every 2 days.

For co-culture with MSCs, cancer cells were detached from culture dish using 0,25% Trypsin-EDTA and resuspended in full RPMI media. UC-MSC cells were prepared as described in Example 1 and then mixed 2:1 (200 000:100 000) with cancer cell line and plated on 24-well plate. Co-culture media was a mixture 1:1 of media for MSC and cancer cells.

The results were analysed by flow cytometry. Before setting up the co-culture in every experiment cancer cells were stained with Cell Trace Violet (Thermo Fisher Scientific) in the 1:100 dilution. In experiments with fluorescent cargo (haemoglon-Alexa488 and ferritin-A488) UC-MSC and cancer cells were differentiated by Cell Trace Violet signal. In experiments with ferritin-doxorubicin all cells in co-culture were additionally stained with Draq7 (BioLegend) in order to track viability. Cells were differentiated by Cell Trace Violet. The results, shown in Figures 2 to 4, demonstrate that the stem cells loaded with the complexes can deliver the complexes to cancer cells and unload them into the cancer cells.

## Claims

1. An isolated targeted delivery system comprising a mesenchymal stem cell (MSC) comprising within said cell a complex of one or more iron-binding proteins and a therapeutic or a diagnostic agent.

2. The isolated targeted delivery system of claim 1, wherein the MSC is selected from the group consisting of an umbilical cord MSC, a bone marrow MSC, an adipose MSC, a placenta MSC, a dental MSC, an amniotic fluid MSC, an endometrium MSC, and an iPSC-derived MSC.

3. The isolated targeted delivery system of claim 1 or claim 2, wherein stem cell is a non-haematopoietic stem cell.

4. The isolated targeted delivery system of any one of claims 1 to 3, wherein the stem cell is an umbilical cord blood MSC or an umbilical cord tissue MSC.

5. The isolated targeted delivery system of any one of claims 1 to 4, wherein the MSC is a human MSC.

6. The isolated targeted delivery system of any of claims 1 to 5, wherein the iron-binding protein is selected from the group consisting of ferritin, preferably heavy (H) type ferritin, light (L) ferritin and/or mitochondrial ferritin; haemoglobin, preferably haemoglobin A, haemoglobin AS, haemoglobin SC, haemoglobin C, haemoglobin D, haemoglobin E, haemoglobin F, haemoglobin H; haemoglobin-haptoglobin complex, hemopexin, transferrin; and lactoferrin.

7. The isolated targeted delivery system of any of claims 1 to 6, wherein the therapeutic agent is selected from the group consisting of a protein, a nucleic acid a chemical non-protein non-nucleic acid compound with a molecular weight of less than 1 kD.

8. The isolated targeted delivery system of any of claims 1 to 6, wherein the therapeutic agent is an immunomodulatory drug, an anticancer drug, and/or a hypoxia-activated prodrug.

9. The isolated targeted delivery system of any of claims 1 to 6, wherein the diagnostic agent is selected from a fluorescent dye, a fluorescence emitting isotope, a radioisotope, a detectable polypeptide or nucleic acid encoding a detectable polypeptide and a contrast agent.

10. The isolated targeted delivery system of any of claims 1 to 6 and 9, wherein the diagnostic agent comprises a chelating agent which forms a complex with divalent or trivalent metal cations.

11. The isolated targeted delivery system according to any of claims 1 to 10, wherein:
(i) the bond(s) between the iron-binding protein(s) and the therapeutic or diagnostic agent comprised in the complex are covalent and/or non-covalent; and/or
(ii) the therapeutic or diagnostic agent comprised in the complex is entrapped/encapsulated by the iron-binding protein or multimers thereof.

12. Method of preparation of the isolated targeted delivery system of claims 1 to 11 comprising steps of
a) providing purified iron-binding protein;
b) covalently or non-covalently linking a therapeutic or diagnostic agent to and/or encapsulating a therapeutic or diagnostic agent in an iron-binding protein;
c) providing an MSC; and
d) incubating the MSC in the presence of the iron-binding protein produced in step b) until the MSC is at least partially loaded with the iron-binding protein produced in step b).

13. The isolated targeted delivery system of any of claims 1-11 or producible with the method of claim 12 for use as a medicament or a diagnostic.

14. A pharmaceutical or diagnostic composition comprising the isolated targeted delivery system of any of claims 1-11 or producible according to the method of claim 12 and a pharmaceutically acceptable carrier and/or suitable excipient(s).

15. The isolated targeted delivery system of any of claims 1-11 or producible according to the method of claim 12 for use in diagnosing, preventing or treating tumours.


SEQUENCE LISTING

<110>  Cellis AG

<120>  Mesenchymal Stem Cell Based Targeted Active Ingredient Delivery
       System

<130>  976-6 EP

<160>  28

<170>  PatentIn version 3.5

<210>  1
<211>  132
<212>  PRT
<213>  Artificial Sequence

<220>
<223>  Minimal consensus sequence of mammalian ferritin H chain


<220>
<221>  MISC_FEATURE
<222>  (5)..(5)
<223>  Xaa at position 5 may be present or absent, if present it means
       any naturally occurring amino acid, preferably Ile

<220>
<221>  MISC_FEATURE
<222>  (6)..(6)
<223>  Xaa at position 6 means any naturally occurring amino acid,
       preferably Asn

<220>
<221>  MISC_FEATURE
<222>  (14)..(14)
<223>  Xaa at position 14 means any naturally occurring amino acid,
       preferably Tyr

<220>
<221>  MISC_FEATURE
<222>  (24)..(24)
<223>  Xaa at position 24 means any naturally occurring amino acid,
       preferably Tyr or Cys, more preferably Tyr

<220>
<221>  MISC_FEATURE
<222>  (66)..(66)
<223>  Xaa at position 66 means any naturally occurring amino acid,
       preferably Phe

<220>
<221>  MISC_FEATURE
<222>  (68)..(68)
<223>  Xaa at position 68 means any naturally occurring amino acid,
       preferably Gln

<220>
<221>  MISC_FEATURE
<222>  (75)..(75)
<223>  Xaa at position 75 means any naturally occurring amino acid,
       preferably Arg or Cys, more preferably Cys

<220>
<221>  MISC_FEATURE
<222>  (90)..(90)
<223>  Xaa at position 90 means any naturally occurring amino acid,
       preferably His

<220>
<221>  MISC_FEATURE
<222>  (94)..(94)
<223>  Xaa at position 94 means any naturally occurring amino acid,
       preferably Ser or Asn, more preferably Asn

<220>
<221>  MISC_FEATURE
<222>  (120)..(120)
<223>  Xaa at position 120 may be present or absent, if present it means
       any naturally occurring amino acid, preferably His or Tyr, more
       preferably His

<220>
<221>  MISC_FEATURE
<222>  (123)..(123)
<223>  Xaa at position 123 means any naturally occurring amino acid,
       preferably Asn or Ser, more preferably Asn

<220>
<221>  MISC_FEATURE
<222>  (128)..(128)
<223>  Xaa at position 128 means any naturally occurring amino acid,
       preferably Ala or Ser, more preferably Ala

<400>  1

Ser Glu Ala Ala Xaa Xaa Arg Gln Ile Asn Leu Glu Leu Xaa Ala Ser
1               5                   10                  15

Tyr Val Tyr Leu Ser Met Ser Xaa Tyr Phe Asp Arg Asp Asp Val Ala
            20                  25                  30

Leu Lys Asn Phe Ala Lys Tyr Phe Leu His Gln Ser His Glu Glu Arg
        35                  40                  45

Glu His Ala Glu Lys Leu Met Lys Leu Gln Asn Gln Arg Gly Gly Arg
    50                  55                  60

Ile Xaa Leu Xaa Asp Ile Lys Lys Pro Asp Xaa Asp Asp Trp Glu Ser
65                  70                  75                  80

Gly Leu Asn Ala Met Glu Cys Ala Leu Xaa Leu Glu Lys Xaa Asn Gln
                85                  90                  95

Ser Leu Leu Glu Leu His Lys Leu Ala Thr Asp Lys Asn Asp Pro His
            100                 105                 110

Leu Cys Asp Phe Ile Glu Thr Xaa Tyr Leu Xaa Glu Gln Val Lys Xaa

115                 120                 125

Ile Lys Glu Leu
    130

<210>  2
<211>  183
<212>  PRT
<213>  Artificial Sequence

<220>
<223>  Consensus sequence of mammalian ferritin H chain

<220>
<221>  MISC_FEATURE
<222>  (6)..(6)
<223>  Xaa at position 6 can be any naturally occurring amino acid,
       preferably Pro

<220>
<221>  MISC_FEATURE
<222>  (14)..(14)
<223>  Xaa at position 14 can be any naturally occurring amino acid,
       preferably His

<220>
<221>  MISC_FEATURE
<222>  (16)..(16)
<223>  Xaa at position 16 can be any naturally occurring amino acid,
       preferably Asp

<220>
<221>  MISC_FEATURE
<222>  (21)..(21)
<223>  Xaa at position 21 may be present or absent, if present it means
       any naturally occurring amino acid, preferably Ile

<220>
<221>  MISC_FEATURE
<222>  (22)..(22)
<223>  Xaa at position 22  means any naturally occurring amino acid,
       preferably Asn

<220>
<221>  MISC_FEATURE
<222>  (30)..(30)
<223>  Xaa at position 30 can be any naturally occurring amino acid,
       preferably Tyr

<220>
<221>  MISC_FEATURE
<222>  (40)..(40)
<223>  Xaa at position 40 can be any naturally occurring amino acid,
       preferably Tyr or Cys, more preferably Tyr

<220>
<221>  MISC_FEATURE
<222>  (82)..(82)
<223>  Xaa at position 82 can be any naturally occurring amino acid,
       preferably Phe

<220>
<221>  MISC_FEATURE
<222>  (84)..(84)
<223>  Xaa at position 84 can be any naturally occurring amino acid,
      preferably Gln

<220>
<221>  MISC_FEATURE
<222>  (91)..(91)
<223>  Xaa at position 91 can be any naturally occurring amino acid,
      preferably Arg or Cys, more preferably Cys

<220>
<221>  MISC_FEATURE
<222>  (106)..(106)
<223>  Xaa at position 106 can be any naturally occurring amino acid,
      preferably His

<220>
<221>  MISC_FEATURE
<222>  (110)..(110)
<223>  Xaa at position 110 can be any naturally occurring amino acid,
      preferably Asn or Ser, more preferably Asn

<220>
<221>  MISC_FEATURE
<222>  (137)..(137)
<223>  Xaa at position 137 can be any naturally occurring amino acid,
      preferably His or Tyr, more preferably His

<220>
<221>  MISC_FEATURE
<222>  (140)..(140)
<223>  Xaa at position 140 can be any naturally occurring amino acid,
      preferably Asn or Ser, more preferably Asn

<220>
<221>  MISC_FEATURE
<222>  (145)..(145)
<223>  Xaa at position 145 can be any naturally occurring amino acid,
      preferably Ala or Ser, more preferably Ala

<220>
<221>  MISC_FEATURE
<222>  (164)..(164)
<223>  Xaa at position 164 can be any naturally occurring amino acid,
      preferably Ala or Ser, more preferably Ser

<220>
<221>  MISC_FEATURE
<222>  (166)..(166)
<223>  Xaa at position 166 can be any naturally occurring amino acid,
      preferably Met or Leu, prerferably Leu

<220>
<221>  MISC_FEATURE
<222>  (178)..(178)
<223>  Xaa at position 178 can be any naturally occurring amino acid,
      preferably Asp or His, more preferably Asp

<220>
<221>  MISC_FEATURE

<222>  (181)..(181)
<223>  Xaa at position 181 is absent or any naturally occurring amino
       acid, preferably Asn

<220>
<221>  MISC_FEATURE
<222>  (182)..(182)
<223>  Xaa at position 182 is absent or any naturally occurring amino
       acid, preferably Glu

<220>
<221>  MISC_FEATURE
<222>  (183)..(183)
<223>  Xaa at position 183 is absent or any naturally occurring amino
       acid, preferably Ser

<400>  2

Met Thr Thr Ala Ser Xaa Ser Gln Val Arg Gln Asn Tyr Xaa Gln Xaa
1               5                   10                  15

Ser Glu Ala Ala Xaa Xaa Arg Gln Ile Asn Leu Glu Leu Xaa Ala Ser
            20                  25                  30

Tyr Val Tyr Leu Ser Met Ser Xaa Tyr Phe Asp Arg Asp Asp Val Ala
        35                  40                  45

Leu Lys Asn Phe Ala Lys Tyr Phe Leu His Gln Ser His Glu Glu Arg
    50                  55                  60

Glu His Ala Glu Lys Leu Met Lys Leu Gln Asn Gln Arg Gly Gly Arg
65                  70                  75                  80

Ile Xaa Leu Xaa Asp Ile Lys Lys Pro Asp Xaa Asp Asp Trp Glu Ser
                85                  90                  95

Gly Leu Asn Ala Met Glu Cys Ala Leu Xaa Leu Glu Lys Xaa Val Asn
            100                 105                 110

Gln Ser Leu Leu Glu Leu His Lys Leu Ala Thr Asp Lys Asn Asp Pro
        115                 120                 125

His Leu Cys Asp Phe Ile Glu Thr Xaa Tyr Leu Xaa Glu Gln Val Lys
    130                 135                 140

Xaa Ile Lys Glu Leu Gly Asp His Val Thr Asn Leu Arg Lys Met Gly
145                 150                 155                 160

Ala Pro Glu Xaa Gly Xaa Ala Glu Tyr Leu Phe Asp Lys His Thr Leu
                165                 170                 175

Gly Xaa Ser Asp Xaa Xaa Xaa

            180

<210>  3
<211>  133
<212>  PRT
<213>  Mus musculus

<400>  3

Ala Glu Ala Ala Ile Asn Arg Gln Ile Asn Leu Glu Leu Tyr Ala Ser
1               5                   10                  15

Tyr Val Tyr Leu Ser Met Ser Cys Tyr Phe Asp Arg Asp Asp Val Ala
            20                  25                  30

Leu Lys Asn Phe Ala Lys Tyr Phe Leu His Gln Ser His Glu Glu Arg
        35                  40                  45

Glu His Ala Glu Lys Leu Met Lys Leu Gln Asn Gln Arg Gly Gly Arg
    50                  55                  60

Ile Phe Leu Gln Asp Ile Lys Lys Pro Asp Arg Asp Asp Trp Glu Ser
65                  70                  75                  80

Gly Leu Asn Ala Met Glu Cys Ala Leu His Leu Glu Lys Ser Val Asn
                85                  90                  95

Gln Ser Leu Leu Glu Leu His Lys Leu Ala Thr Asp Lys Asn Asp Pro
            100                 105                 110

His Leu Cys Asp Phe Ile Glu Thr Tyr Tyr Leu Ser Glu Gln Val Lys
        115                 120                 125

Ser Ile Lys Glu Leu
    130


<210>  4
<211>  180
<212>  PRT
<213>  Mus musculus

<400>  4

Met Thr Thr Ala Ser Pro Ser Gln Val Arg Gln Asn Tyr His Gln Asp
1               5                   10                  15

Ala Glu Ala Ala Ile Asn Arg Gln Ile Asn Leu Glu Leu Tyr Ala Ser
            20                  25                  30

Tyr Val Tyr Leu Ser Met Ser Cys Tyr Phe Asp Arg Asp Asp Val Ala
        35                  40                  45

Leu Lys Asn Phe Ala Lys Tyr Phe Leu His Gln Ser His Glu Glu Arg
    50                  55                  60

Glu His Ala Glu Lys Leu Met Lys Leu Gln Asn Gln Arg Gly Gly Arg
65                  70                  75                  80

Ile Phe Leu Gln Asp Ile Lys Lys Pro Asp Arg Asp Asp Trp Glu Ser
                85                  90                  95

Gly Leu Asn Ala Met Glu Cys Ala Leu His Leu Glu Lys Ser Val Asn
            100                 105                 110

Gln Ser Leu Leu Glu Leu His Lys Leu Ala Thr Asp Lys Asn Asp Pro
        115                 120                 125

His Leu Cys Asp Phe Ile Glu Thr Tyr Tyr Leu Ser Glu Gln Val Lys
    130                 135                 140

Ser Ile Lys Glu Leu Gly Asp His Val Thr Asn Leu Arg Lys Met Gly
145                 150                 155                 160

Ala Pro Glu Ala Gly Met Ala Glu Tyr Leu Phe Asp Lys His Thr Leu
                165                 170                 175

Gly His Gly Asp
            180

<210>  5
<211>  132
<212>  PRT
<213>  Homo sapiens

<400>  5

Ser Glu Ala Ala Asn Arg Gln Ile Asn Leu Glu Leu Tyr Ala Ser Tyr
1               5                   10                  15

Val Tyr Leu Ser Met Ser Tyr Tyr Phe Asp Arg Asp Asp Val Ala Leu
            20                  25                  30

Lys Asn Phe Ala Lys Tyr Phe Leu His Gln Ser His Glu Glu Arg Glu
        35                  40                  45

His Ala Glu Lys Leu Met Lys Leu Gln Asn Gln Arg Gly Gly Arg Ile
    50                  55                  60

Phe Leu Gln Asp Ile Lys Lys Pro Asp Cys Asp Asp Trp Glu Ser Gly
65                  70                  75                  80

Leu Asn Ala Met Glu Cys Ala Leu His Leu Glu Lys Asn Val Asn Gln
                85                  90                  95

Ser Leu Leu Glu Leu His Lys Leu Ala Thr Asp Lys Asn Asp Pro His
            100                 105                 110

Leu Cys Asp Phe Ile Glu Thr His Tyr Leu Asn Glu Gln Val Lys Ala
        115                 120                 125

Ile Lys Glu Leu
    130

<210>  6
<211>  179
<212>  PRT
<213>  Homo sapiens

<400>  6

Met Thr Thr Ala Ser Thr Ser Gln Val Arg Gln Asn Tyr His Gln Asp
1               5                   10                  15

Ser Glu Ala Ala Asn Arg Gln Ile Asn Leu Glu Leu Tyr Ala Ser Tyr
            20                  25                  30

Val Tyr Leu Ser Met Ser Tyr Tyr Phe Asp Arg Asp Asp Val Ala Leu
        35                  40                  45

Lys Asn Phe Ala Lys Tyr Phe Leu His Gln Ser His Glu Glu Arg Glu
    50                  55                  60

His Ala Glu Lys Leu Met Lys Leu Gln Asn Gln Arg Gly Gly Arg Ile
65                  70                  75                  80

Phe Leu Gln Asp Ile Lys Lys Pro Asp Cys Asp Asp Trp Glu Ser Gly
                85                  90                  95

Leu Asn Ala Met Glu Cys Ala Leu His Leu Glu Lys Asn Val Asn Gln
            100                 105                 110

Ser Leu Leu Glu Leu His Lys Leu Ala Thr Asp Lys Asn Asp Pro His
        115                 120                 125

Leu Cys Asp Phe Ile Glu Thr His Tyr Leu Asn Glu Gln Val Lys Ala
    130                 135                 140

Ile Lys Glu Leu Gly Asp His Val Thr Asn Leu Arg Lys Met Gly Ala
145                 150                 155                 160

Pro Glu Ser Gly Leu Ala Glu Tyr Leu Phe Asp Lys His Thr Leu Gly
                165                 170                 175

Asp Ser Asp

<210>  7
<211>  182
<212>  PRT
<213>  Artificial Sequence

<220>
<223>  Consensus sequence of mammalian ferritin H chains 2nd version

<220>
<221>  MISC_FEATURE
<222>  (6)..(6)
<223>  Xaa at position 6 can be any naturally occurring amino acid,
       preferably Pro

<220>
<221>  MISC_FEATURE
<222>  (14)..(14)
<223>  Xaa at position 14 can be any naturally occurring amino acid,
       preferably His

<220>
<221>  MISC_FEATURE
<222>  (16)..(16)
<223>  Xaa at position 16 can be any naturally occurring amino acid,
       preferably Asp

<220>
<221>  MISC_FEATURE
<222>  (21)..(21)
<223>  Xaa at position 21 may be present or absent, if present it means
       any naturally occurring amino acid, preferably Ile

<220>
<221>  MISC_FEATURE
<222>  (29)..(29)
<223>  Xaa at position 29 can be any naturally occurring amino acid,
       preferably Tyr

<220>
<221>  MISC_FEATURE
<222>  (81)..(81)
<223>  Xaa at position 81 can be any naturally occurring amino acid,
       preferably Phe

<220>
<221>  MISC_FEATURE
<222>  (83)..(83)
<223>  Xaa at position 83 can be any naturally occurring amino acid,
       preferably Gln

<220>
<221>  MISC_FEATURE

<222>  (105)..(105)
<223>  Xaa at position 105 can be any naturally occurring amino acid,
       preferably His

<220>
<221>  MISC_FEATURE
<222>  (144)..(144)
<223>  Xaa at position 144 can be any naturally occurring amino acid,
       preferably Ala or Ser, more preferably Ala

<220>
<221>  MISC_FEATURE
<222>  (180)..(180)
<223>  Xaa at position 180 is absent or any naturally occuring amino
       acid, preferably Asn

<220>
<221>  MISC_FEATURE
<222>  (181)..(181)
<223>  Xaa at position 181 is absent or any naturally occuring amino
       acid, preferably Glu

<220>
<221>  MISC_FEATURE
<222>  (182)..(182)
<223>  Xaa at position 182 is absent or any naturally occuring amino
       acid, preferably Ser

<400>  7

Met Thr Thr Ala Ser Xaa Ser Gln Val Arg Gln Asn Tyr Xaa Gln Xaa
1               5                   10                  15

Ser Glu Ala Ala Xaa Arg Gln Ile Asn Leu Glu Leu Xaa Ala Ser Tyr
            20                  25                  30

Val Tyr Leu Ser Met Ser Tyr Tyr Phe Asp Arg Asp Asp Val Ala Leu
        35                  40                  45

Lys Asn Phe Ala Lys Tyr Phe Leu His Gln Ser His Glu Glu Arg Glu
    50                  55                  60

His Ala Glu Lys Leu Met Lys Leu Gln Asn Gln Arg Gly Gly Arg Ile
65                  70                  75                  80

Xaa Leu Xaa Asp Ile Lys Lys Pro Asp Cys Asp Asp Trp Glu Ser Gly
                85                  90                  95

Leu Asn Ala Met Glu Cys Ala Leu Xaa Leu Glu Lys Asn Val Asn Gln
            100                 105                 110

Ser Leu Leu Glu Leu His Lys Leu Ala Thr Asp Lys Asn Asp Pro His
        115                 120                 125

Leu Cys Asp Phe Ile Glu Thr His Tyr Leu Asn Glu Gln Val Lys Xaa

130                 135                 140

Ile Lys Glu Leu Gly Asp His Val Thr Asn Leu Arg Lys Met Gly Ala
145                 150                 155                 160

Pro Glu Ser Gly Leu Ala Glu Tyr Leu Phe Asp Lys His Thr Leu Gly
                165                 170                 175

Asp Ser Asp Xaa Xaa Xaa
            180

<210>  8
<211>  150
<212>  PRT
<213>  Artificial Sequence

<220>
<223>  Minimal consensus sequence of mammalian ferritin L chain

<220>
<221>  MISC_FEATURE
<222>  (5)..(5)
<223>  Xaa at position 5 can be any naturally occurring amino acid,
       preferably Asp or Glu, more preferably Asp

<220>
<221>  MISC_FEATURE
<222>  (12)..(12)
<223>  Xaa at position 12 can be any nnaturally occurring amino acid,
       preferably Arg or Ser, more preferably Ser

<220>
<221>  MISC_FEATURE
<222>  (17)..(17)
<223>  Xaa at position 17 can be any naturally occurring amino acid,
       preferably Ser or Arg, more preferably Ser

<220>
<221>  MISC_FEATURE
<222>  (19)..(19)
<223>  Xaa at position 19 can be any naturally occurring amino acid,
       preferably Arg or Gln, more preferably Gln

<220>
<221>  MISC_FEATURE
<222>  (29)..(29)
<223>  Xaa at position 29 can be any naturally occurring amino acid,
       preferably Phe

<220>
<221>  MISC_FEATURE
<222>  (30)..(30)
<223>  Xaa at position 30 can be any naturally occurring amino acid,
       preferably Tyr or Phe, more preferably Tyr

<220>
<221>  MISC_FEATURE
<222>  (42)..(42)

<223>  Xaa at position 42 can be any naturally occurring amino acid,
      preferably Ser or Gly, more preferably Ser

<220>
<221>  MISC_FEATURE
<222>  (56)..(56)
<223>  Xaa at position 56 can be any naturally occurirng amino acid,
      preferably Ala or Tyr, more preferably Tyr

<220>
<221>  MISC_FEATURE
<222>  (61)..(61)
<223>  Xaa at position 61 can be any naturally occurring amino acid,
      preferably Glu or Lys, more preferably Lys

<220>
<221>  MISC_FEATURE
<222>  (62)..(62)
<223>  Xaa at position 62 can be any naturally occurring amino acid,
      preferably Met or Phe, more preferably Met

<220>
<221>  MISC_FEATURE
<222>  (65)..(65)
<223>  Xaa at position 65 can be any naturally occurring amino acid,
      preferably Asp or Gln, more preferably Gln

<220>
<221>  MISC_FEATURE
<222>  (75)..(75)
<223>  Xaa at position 75 can be any naturally occurring amino acid,
      preferably Ile or Val, more preferably Ile

<220>
<221>  MISC_FEATURE
<222>  (76)..(76)
<223>  Xaa at position 76 can be any naturally occurring amino acid,
      preferably Lys or Gln, more preferably Lys

<220>
<221>  MISC_FEATURE
<222>  (79)..(79)
<223>  Xaa at position 79 can be any naturally occurring amino acid,
      preferably Ala or Ser, more preferably Ala

<220>
<221>  MISC_FEATURE
<222>  (80)..(80)
<223>  Xaa at position 80 can be any naturally occurring amino acid,
      preferably Glu or Gln, more preferably Gln

<220>
<221>  MISC_FEATURE
<222>  (87)..(87)
<223>  Xaa at position 87 can be any naturally occurring amino acid,
      preferably Pro or Gln, more preferably Pro

<220>
<221>  MISC_FEATURE
<222>  (88)..(88)
<223>  Xaa at position 88 can be any naturally occurring amino acid,
      preferably Glu or Asp, more preferably Asp

<220>
<221>  MISC_FEATURE
<222>  (91)..(91)
<223>  Xaa at position 91 can be any naturally occurring amino acid,
      preferably Glu or Lys, more preferably Lys

<220>
<221>  MISC_FEATURE
<222>  (94)..(94)
<223>  Xaa at position 94 can be any naturally occurring amino acid,
      preferably Met or Leu, more preferably Leu

<220>
<221>  MISC_FEATURE
<222>  (96)..(96)
<223>  Xaa at position 96 can be any naturally occurring amino acid,
      preferably Met or Leu, more preferably Met

<220>
<221>  MISC_FEATURE
<222>  (99)..(99)
<223>  Xaa at position 99 can be any naturally occurring amino acid,
      preferably Lys or Asn, preferably Lys

<220>
<221>  MISC_FEATURE
<222>  (115)..(115)
<223>  Xaa at position 115 can be any naturally occurring amino acid,
      preferably Thr or Ala, more preferably Thr

<220>
<221>  MISC_FEATURE
<222>  (119)..(119)
<223>  Xaa at position 119 can be any naturally occurring amino acid,
      preferably Leu

<220>
<221>  MISC_FEATURE
<222>  (125)..(125)
<223>  Xaa at position 125 can be any naturally occurring amino acid,
      preferably Ser or Thr, more preferably Thr

<220>
<221>  MISC_FEATURE
<222>  (127)..(127)
<223>  Xaa at position 127 can be any naturally occurring amino acid,
      preferably Tyr or Phe, more preferably Phe

<220>
<221>  MISC_FEATURE
<222>  (130)..(130)
<223>  Xaa at position 130 can be any naturally occurring amino acid,
      preferably Lys or Glu, more preferably Glu

<220>
<221>  MISC_FEATURE
<222>  (140)..(140)
<223>  Xaa at position 140 can be any naturally occurring amino acid,
      preferably Asp or Asn, more preferably Asp

<220>
<221>  MISC_FEATURE
<222>  (146)..(146)

<223>  Xaa at position 146 can be any naturally occurring amino acid,
       preferably Arg or His, more preferably His

<220>
<221>  MISC_FEATURE
<222>  (148)..(148)
<223>  Xaa at position 148 can be any naturally occurring amino acid,
       preferably Leu or Val, more preferably Leu

<400>  8

Asn Tyr Ser Thr Xaa Val Glu Ala Ala Val Asn Xaa Leu Val Asn Leu
1               5                   10                  15

Xaa Leu Xaa Ala Ser Tyr Thr Tyr Leu Ser Leu Gly Xaa Xaa Phe Asp
            20                  25                  30

Arg Asp Asp Val Ala Leu Glu Gly Val Xaa His Phe Phe Arg Glu Leu
        35                  40                  45

Ala Glu Glu Lys Arg Glu Gly Xaa Glu Arg Leu Leu Xaa Xaa Gln Asn
    50                  55                  60

Xaa Arg Gly Gly Arg Ala Leu Phe Gln Asp Xaa Xaa Lys Pro Xaa Xaa
65                  70                  75                  80

Asp Glu Trp Gly Lys Thr Xaa Xaa Ala Met Xaa Ala Ala Xaa Ala Xaa
                85                  90                  95

Glu Lys Xaa Leu Asn Gln Ala Leu Leu Asp Leu His Ala Leu Gly Ser
            100                 105                 110

Ala Arg Xaa Asp Pro His Xaa Cys Asp Phe Leu Glu Xaa His Xaa Leu
        115                 120                 125

Asp Xaa Glu Val Lys Leu Ile Lys Lys Met Gly Xaa His Leu Thr Asn
    130                 135                 140

Leu Xaa Arg Xaa Ala Gly
145                 150

<210>  9
<211>  183
<212>  PRT
<213>  Artificial Sequence

<220>
<223>  Consensus sequence of mammalian ferritin L chain

<220>
<221>  MISC_FEATURE
<222>  (12)..(12)

<223>  Xaa at position 12 can be any naturally occurring amino acid,
      preferably Asp or Glu, more preferably Asp

<220>
<221>  MISC_FEATURE
<222>  (19)..(19)
<223>  Xaa at position 19 can be any naturally occurring amino acid,
      preferably Ser or Arg, more preferably Ser

<220>
<221>  MISC_FEATURE
<222>  (24)..(24)
<223>  Xaa at position 24 can be any naturally occurring amino acid,
      preferably Ser or Arg, more preferably Ser

<220>
<221>  MISC_FEATURE
<222>  (26)..(26)
<223>  Xaa at position 26 can be any naturally occurring amino acid,
      preferably Arg or Gln, more preferably Gln

<220>
<221>  MISC_FEATURE
<222>  (36)..(36)
<223>  Xaa at position 36 can be any naturally occurring amino acid,
      preferably Phe

<220>
<221>  MISC_FEATURE
<222>  (37)..(37)
<223>  Xaa at position 37 can be any naturally occurring amino acid,
      preferably Tyr or Phe, more preferably Tyr

<220>
<221>  MISC_FEATURE
<222>  (49)..(49)
<223>  Xaa at position 47 can be any naturally occurring amino acid,
      preferably Ser or Gly, more preferably Ser

<220>
<221>  MISC_FEATURE
<222>  (63)..(63)
<223>  Xaa at position 63 can be any naturally occurring amino acid,
      preferably Ala or Tyr, more preferably Tyr

<220>
<221>  MISC_FEATURE
<222>  (68)..(68)
<223>  Xaa at position 68 can be any naturally occurring amino acid,
      preferably Glu or Lys, more preferably Lys

<220>
<221>  MISC_FEATURE
<222>  (69)..(69)
<223>  Xaa at position 69 can be any naturally occurring amino acid,
      preferably Met or Phe, more preferably Met

<220>
<221>  MISC_FEATURE
<222>  (72)..(72)
<223>  Xaa at position 72 can be any naturally occurring amino acid,
      preferably Asp or Gln, more preferably Gln

<220>
<221>  MISC_FEATURE
<222>  (82)..(82)
<223>  Xaa at position 82 can be any naturally occurring amino acid,
      preferably Ile or Val, more preferably Ile

<220>
<221>  MISC_FEATURE
<222>  (83)..(83)
<223>  Xaa at position 83 can be any naturally occurring amino acid,
      preferably Lys or Gln, more preferably Lys

<220>
<221>  MISC_FEATURE
<222>  (86)..(86)
<223>  Xaa at position 86 can be any naturally occurring amino acid,
      preferably Ala or Ser, more preferably Ala

<220>
<221>  MISC_FEATURE
<222>  (87)..(87)
<223>  Xaa at position 87 can be any naturally occurring amino acid,
      preferably Glu or Gln, more preferably Gln

<220>
<221>  MISC_FEATURE
<222>  (94)..(94)
<223>  Xaa at position 94 can be any naturally occurring amino acid,
      preferably Pro or Gln, more preferably Pro

<220>
<221>  MISC_FEATURE
<222>  (95)..(95)
<223>  Xaa at position 95 can be any naturally occurring amino acid,
      preferably Glu or Asp, more preferably Asp

<220>
<221>  MISC_FEATURE
<222>  (98)..(98)
<223>  Xaa at position 98 can be any naturally occurring amino acid,
      preferably Glu or Lys, more preferably Lys

<220>
<221>  MISC_FEATURE
<222>  (101)..(101)
<223>  Xaa at position 101 can be any naturally occurring amino acid,
      preferably Met or Leu, more preferably Leu

<220>
<221>  MISC_FEATURE
<222>  (103)..(103)
<223>  Xaa at position 103 can be any naturally occurring amino acid,
      preferably Met or Leu, more preferably Met

<220>
<221>  MISC_FEATURE
<222>  (106)..(106)
<223>  Xaa at position 106 can be any naturally occurring amino acid,
      preferably Lys or Asn, preferably Lys

<220>
<221>  misc_feature
<222>  (122)..(122)

<223>  Xaa can be any naturally occurring amino acid

<220>
<221>  MISC_FEATURE
<222>  (126)..(126)
<223>  Xaa at position 126 can be any naturally occurring  amino acid,
      preferably Leu

<220>
<221>  MISC_FEATURE
<222>  (132)..(132)
<223>  Xaa at position 132 can be any naturally occurring amino acid,
      preferably Ser or Thr, more preferably Thr

<220>
<221>  MISC_FEATURE
<222>  (134)..(134)
<223>  Xaa at position 134 can be any naturally occurring amino acid,
      preferably Tyr or Phe, more preferably Phe

<220>
<221>  MISC_FEATURE
<222>  (137)..(137)
<223>  Xaa at position 137 can be any naturally occurring amino acid,
      preferably Lys or Glu, more preferably Glu

<220>
<221>  MISC_FEATURE
<222>  (147)..(147)
<223>  Xaa at position 147 can be any naturally occurring amino acid,
      preferably Asp or Asn, more preferably Asp

<220>
<221>  MISC_FEATURE
<222>  (153)..(153)
<223>  Xaa at position 153 can be any naturally occurring amino acid,
      preferably Arg or His, more preferably His

<220>
<221>  MISC_FEATURE
<222>  (155)..(155)
<223>  Xaa at position 155 can be any naturally occurring  amino acid,
      preferably Leu or Val, more preferably Leu

<220>
<221>  MISC_FEATURE
<222>  (161)..(161)
<223>  Xaa at position 161 can be absent or any naturally occurring
      amino acid, preferably Ala

<220>
<221>  MISC_FEATURE
<222>  (163)..(163)
<223>  Xaa at position 163 can be absent or any naturally occurring
      amino acid, preferably Thr

<220>
<221>  MISC_FEATURE
<222>  (166)..(166)
<223>  Xaa at position 166 can be absent or any naturally occurring
      amino acid, preferably Pro

<220>

<221>  MISC_FEATURE
<222>  (168)..(168)
<223>  Xaa at position 168 can be any naturally occurring amino acid,
       preferably Gly or Ala, more preferably Ala

<400>  9

Met Thr Ser Gln Ile Arg Gln Asn Tyr Ser Thr Xaa Val Glu Ala Ala
1               5                   10                  15

Val Asn Xaa Leu Val Asn Leu Xaa Leu Xaa Ala Ser Tyr Thr Tyr Leu
            20                  25                  30

Ser Leu Gly Xaa Xaa Phe Asp Arg Asp Asp Val Ala Leu Glu Gly Val
        35                  40                  45

Xaa His Phe Phe Arg Glu Leu Ala Glu Glu Lys Arg Glu Gly Xaa Glu
    50                  55                  60

Arg Leu Leu Xaa Xaa Gln Asn Xaa Arg Gly Gly Arg Ala Leu Phe Gln
65                  70                  75                  80

Asp Xaa Xaa Lys Pro Xaa Xaa Asp Glu Trp Gly Lys Thr Xaa Xaa Ala
                85                  90                  95

Met Xaa Ala Ala Xaa Ala Xaa Glu Lys Xaa Leu Asn Gln Ala Leu Leu
            100                 105                 110

Asp Leu His Ala Leu Gly Ser Ala Arg Xaa Asp Pro His Xaa Cys Asp
        115                 120                 125

Phe Leu Glu Xaa His Xaa Leu Asp Xaa Glu Val Lys Leu Ile Lys Lys
    130                 135                 140

Met Gly Xaa His Leu Thr Asn Leu Xaa Arg Xaa Ala Gly Pro Gln Pro
145                 150                 155                 160

Xaa Gln Xaa Gly Val Xaa Gln Xaa Ser Leu Gly Glu Tyr Leu Phe Glu
                165                 170                 175

Arg Leu Thr Leu Lys His Asp
            180

<210>  10
<211>  150
<212>  PRT
<213>  Mus musculus

<220>
<221>  MISC_FEATURE

<222>  (12)..(12)
<223>  Xaa at position 5 can be any naturally occuring amino acid,
       preferably Asp or Glu, more preferably Asp

<400>  10

Asn Tyr Ser Thr Glu Val Glu Ala Ala Val Asn Arg Leu Val Asn Leu
1               5                   10                  15

His Leu Arg Ala Ser Tyr Thr Tyr Leu Ser Leu Gly Phe Phe Phe Asp
            20                  25                  30

Arg Asp Asp Val Ala Leu Glu Gly Val Gly His Phe Phe Arg Glu Leu
        35                  40                  45

Ala Glu Glu Lys Arg Glu Gly Ala Glu Arg Leu Leu Glu Phe Gln Asn
    50                  55                  60

Asp Arg Gly Gly Arg Ala Leu Phe Gln Asp Val Gln Lys Pro Ser Gln
65                  70                  75                  80

Asp Glu Trp Gly Lys Thr Gln Glu Ala Met Glu Ala Ala Leu Ala Met
                85                  90                  95

Glu Lys Asn Leu Asn Gln Ala Leu Leu Asp Leu His Ala Leu Gly Ser
            100                 105                 110

Ala Arg Ala Asp Pro His Leu Cys Asp Phe Leu Glu Ser His Tyr Leu
        115                 120                 125

Asp Lys Glu Val Lys Leu Ile Lys Lys Met Gly Asn His Leu Thr Asn
    130                 135                 140

Leu Arg Arg Val Ala Gly
145                 150

<210>  11

<400>  11
000

<210>  12
<211>  149
<212>  PRT
<213>  Homo sapiens

<400>  12

Asn Tyr Ser Thr Asp Val Glu Ala Ala Val Asn Ser Leu Val Asn Leu
1               5                   10                  15

Tyr Leu Gln Ala Ser Tyr Thr Tyr Leu Ser Leu Gly Phe Tyr Phe Asp

20                  25                  30

Arg Asp Asp Val Ala Leu Glu Gly Val Ser His Phe Phe Arg Glu Leu
        35                  40                  45

Ala Glu Glu Lys Arg Glu Gly Tyr Glu Arg Leu Leu Lys Met Gln Asn
    50                  55                  60

Gln Arg Gly Gly Arg Ala Leu Phe Gln Asp Ile Lys Lys Pro Ala Glu
65                  70                  75                  80

Asp Glu Trp Gly Lys Thr Pro Asp Ala Met Lys Ala Ala Met Ala Leu
                85                  90                  95

Glu Lys Lys Leu Asn Gln Ala Leu Leu Asp Leu His Ala Leu Gly Ser
            100                 105                 110

Ala Arg Thr Asp Pro His Leu Cys Asp Phe Leu Glu Thr His Phe Leu
        115                 120                 125

Asp Glu Glu Val Lys Leu Ile Lys Lys Met Gly Asp His Leu Thr Asn
    130                 135                 140

Leu His Arg Leu Gly
145

<210>  13
<211>  175
<212>  PRT
<213>  Homo sapiens

<400>  13

<210>  14
<211>  183
<212>  PRT
<213>  Artificial Sequence

<220>
<223>  Consensus seqeuence of mammaliam ferritin L chain 2nd version


<220>
<221>  MISC_FEATURE
<222>  (36)..(36)
<223>  Xaa at position 36 can be any naturally occuring amino acid,
       preferably Phe

<220>
<221>  MISC_FEATURE
<222>  (37)..(37)
<223>  Xaa at position 37 can be any naturally occuring amino acid,
       preferably Tyr or Phe, more preferably Tyr

<220>
<221>  MISC_FEATURE
<222>  (94)..(94)
<223>  Xaa at position 94 can be any naturally occurring  amino acid,
       preferably Pro or Gln, more preferably Pro

<220>
<221>  MISC_FEATURE
<222>  (126)..(126)
<223>  Xaa at position 126 can be any naturally occurring  amino acid,
       preferably Leu

<220>
<221>  MISC_FEATURE
<222>  (137)..(137)
<223>  Xaa at position 137 can be any naturally occurring  amino acid,
       preferably Lys or Glu, more preferably Glu

<220>

<221>  MISC_FEATURE
<222>  (147)..(147)
<223>  Xaa at position 147 can be any naturally occurring  amino acid,
       preferably Asp or Asn, more preferably Asp

<220>
<221>  misc_feature
<222>  (161)..(161)
<223>  Xaa can be any naturally occurring amino acid

<220>
<221>  MISC_FEATURE
<222>  (163)..(163)
<223>  Xaa at position 163 can be absent or any naturally occurring
       amino acid, preferably Thr

<220>
<221>  MISC_FEATURE
<222>  (166)..(166)
<223>  Xaa at position 166 can be absent or any naturally occurring
       amino acid, preferably Pro

<220>
<221>  MISC_FEATURE
<222>  (168)..(168)
<223>  Xaa at position 168 can be any naturally occurring  amino acid,
       preferably Gly or Ala, more preferably Ala

<400>  14

Met Thr Ser Gln Ile Arg Gln Asn Tyr Ser Thr Glu Val Glu Ala Ala
1               5                   10                  15

Val Asn Arg Leu Val Asn Leu His Leu Arg Ala Ser Tyr Thr Tyr Leu
            20                  25                  30

Ser Leu Gly Xaa Xaa Phe Asp Arg Asp Asp Val Ala Leu Glu Gly Val
        35                  40                  45

Gly His Phe Phe Arg Glu Leu Ala Glu Glu Lys Arg Glu Gly Ala Glu
    50                  55                  60

Arg Leu Leu Lys Leu Gln Asn Gln Arg Gly Gly Arg Ala Leu Phe Gln
65                  70                  75                  80

Asp Val Gln Lys Pro Ala Gln Asp Glu Trp Gly Lys Thr Xaa Glu Ala
                85                  90                  95

Met Glu Ala Ala Leu Ala Leu Glu Lys Asn Leu Asn Gln Ala Leu Leu
            100                 105                 110

Asp Leu His Ala Leu Gly Ser Ala Arg Thr Asp Pro His Xaa Cys Asp
        115                 120                 125

Phe Leu Glu Asn His Phe Leu Asp Xaa Glu Val Lys Leu Ile Lys Lys

130                 135                 140

Met Gly Xaa His Leu Thr Asn Leu Arg Arg Leu Ala Gly Pro Gln Pro
145                 150                 155                 160

Xaa Gln Xaa Gly Val Xaa Gln Xaa Ser Leu Gly Glu Tyr Leu Phe Glu
                165                 170                 175

Arg Leu Thr Leu Lys His Asp
            180

<210>  15
<211>  117
<212>  PRT
<213>  Artificial Sequence

<220>
<223>  Minimal consensus sequence of mammalian haemoglobin alpha chain

<220>
<221>  MISC_FEATURE
<222>  (5)..(5)
<223>  Xaa can be any naturally occurring amino acid, preferably Ala

<220>
<221>  MISC_FEATURE
<222>  (9)..(9)
<223>  Xaa can be any naturally occurring amino acid, preferably Gly

<220>
<221>  MISC_FEATURE
<222>  (15)..(15)
<223>  Xaa can be any naturally occurring amino acid, preferably Arg

<220>
<221>  MISC_FEATURE
<222>  (21)..(21)
<223>  Xaa can be any naturally occurring amino acid, preferably Pro

<220>
<221>  MISC_FEATURE
<222>  (22)..(22)
<223>  Xaa can be any naturally occurring amino acid, preferably Thr

<220>
<221>  MISC_FEATURE
<222>  (38)..(38)
<223>  Xaa can be any naturally occurring amino acid, preferably Gln

<220>
<221>  MISC_FEATURE
<222>  (41)..(41)
<223>  Xaa can be any naturally occurring amino acid, preferably Gly

<220>
<221>  MISC_FEATURE
<222>  (48)..(48)
<223>  Xaa can be any naturally occurring amino acid, preferably Asp

<220>
<221>  MISC_FEATURE
<222>  (49)..(49)
<223>  Xaa can be any naturally occurring amino acid, preferably Ala

<220>
<221>  MISC_FEATURE
<222>  (55)..(55)
<223>  Xaa can be any naturally occurring amino acid, preferably Ala

<220>
<221>  MISC_FEATURE
<222>  (59)..(59)
<223>  Xaa can be any naturally occurring amino acid, preferably Aap

<220>
<221>  MISC_FEATURE
<222>  (60)..(60)
<223>  Xaa can be any naturally occurring amino acid, preferably Met

<220>
<221>  MISC_FEATURE
<222>  (68)..(68)
<223>  Xaa can be any naturally occurring amino acid, preferably Ser

<220>
<221>  MISC_FEATURE
<222>  (76)..(76)
<223>  Xaa can be any naturally occurring amino acid, preferably Arg

<220>
<221>  MISC_FEATURE
<222>  (79)..(79)
<223>  Xaa can be any naturally occurring amino acid, preferably Pro

<220>
<221>  MISC_FEATURE
<222>  (90)..(90)
<223>  Xaa can be any naturally occurring amino acid, preferably Leu

<220>
<221>  MISC_FEATURE
<222>  (91)..(91)
<223>  Xaa can be any naturally occurring amino acid, preferably Val

<220>
<221>  MISC_FEATURE
<222>  (94)..(94)
<223>  Xaa can be any naturally occurring amino acid, preferably Ala

<220>
<221>  MISC_FEATURE
<222>  (102)..(102)
<223>  Xaa can be any naturally occurring amino acid, preferably Thr

<220>
<221>  MISC_FEATURE
<222>  (108)..(108)
<223>  Xaa can be any naturally occurring amino acid, preferably Ser

<220>
<221>  MISC_FEATURE

<222>  (117)..(117)
<223>  Xaa can be any naturally occurring amino acid, preferably Ser

<400>  15

Val Gly Ala His Xaa Gly Glu Tyr Xaa Ala Glu Ala Leu Glu Xaa Met
1               5                   10                  15

Phe Leu Ser Phe Xaa Xaa Thr Lys Thr Tyr Phe Pro His Phe Asp Leu
            20                  25                  30

Ser His Gly Ser Ala Xaa Val Lys Xaa His Gly Lys Lys Val Ala Xaa
        35                  40                  45

Xaa Leu Thr Asn Ala Val Xaa His Val Asp Xaa Xaa Pro Asn Ala Leu
    50                  55                  60

Ser Ala Leu Xaa Asp Leu His Ala His Lys Leu Xaa Val Asp Xaa Val
65                  70                  75                  80

Asn Phe Lys Leu Leu Ser His Cys Leu Xaa Xaa Thr Leu Xaa Ala His
                85                  90                  95

Leu Pro Ala Glu Phe Xaa Pro Ala Val His Ala Xaa Leu Asp Lys Phe
            100                 105                 110

Leu Ala Ser Val Xaa
        115

<210>  16
<211>  142
<212>  PRT
<213>  Artificial Sequence

<220>
<223>  Consensus sequence of mammalian alpha chain haemoglobins

<220>
<221>  MISC_FEATURE
<222>  (1)..(1)
<223>  Xaa can be any naturally occurring amino acid, preferably Met

<220>
<221>  MISC_FEATURE
<222>  (2)..(2)
<223>  Xaa can be any naturally occurring amino acid, preferably Val

<220>
<221>  MISC_FEATURE
<222>  (14)..(14)
<223>  Xaa can be any naturally occurring amino acid, preferably Ala

<220>
<221>  MISC_FEATURE

<222>  (17)..(17)
<223>  Xaa can be any naturally occurring amino acid, preferably Lys

<220>
<221>  MISC_FEATURE
<222>  (22)..(22)
<223>  Xaa can be any naturally occurring amino acid, preferably Ala

<220>
<221>  MISC_FEATURE
<222>  (26)..(26)
<223>  Xaa can be any naturally occurring amino acid, preferably Gly

<220>
<221>  MISC_FEATURE
<222>  (32)..(32)
<223>  Xaa can be any naturally occurring amino acid, preferably Arg

<220>
<221>  MISC_FEATURE
<222>  (38)..(38)
<223>  Xaa can be any naturally occurring amino acid, preferably Pro

<220>
<221>  MISC_FEATURE
<222>  (39)..(39)
<223>  Xaa can be any naturally occurring amino acid, preferably Thr

<220>
<221>  MISC_FEATURE
<222>  (55)..(55)
<223>  Xaa can be any naturally occurring amino acid, preferably Gln

<220>
<221>  MISC_FEATURE
<222>  (58)..(58)
<223>  Xaa can be any naturally occurring amino acid, preferably Gly

<220>
<221>  MISC_FEATURE
<222>  (65)..(65)
<223>  Xaa can be any naturally occurring amino acid, preferably Asp

<220>
<221>  MISC_FEATURE
<222>  (66)..(66)
<223>  Xaa can be any naturally occurring amino acid, preferably Ala

<220>
<221>  MISC_FEATURE
<222>  (72)..(72)
<223>  Xaa can be any naturally occurring amino acid, preferably Ala

<220>
<221>  MISC_FEATURE
<222>  (76)..(76)
<223>  Xaa can be any naturally occurring amino acid, preferably Asp

<220>
<221>  MISC_FEATURE
<222>  (77)..(77)
<223>  Xaa can be any naturally occurring amino acid, preferably Met

<220>
<221>  MISC_FEATURE
<222>  (85)..(85)
<223>  Xaa can be any naturally occurring amino acid, preferably Ser

<220>
<221>  MISC_FEATURE
<222>  (93)..(93)
<223>  Xaa can be any naturally occurring amino acid, preferably Arg

<220>
<221>  MISC_FEATURE
<222>  (96)..(96)
<223>  Xaa can be any naturally occurring amino acid, preferably Pro

<220>
<221>  MISC_FEATURE
<222>  (107)..(107)
<223>  Xaa can be any naturally occurring amino acid, preferably Leu

<220>
<221>  MISC_FEATURE
<222>  (108)..(108)
<223>  Xaa can be any naturally occurring amino acid, preferably Val

<220>
<221>  MISC_FEATURE
<222>  (111)..(111)
<223>  Xaa can be any naturally occurring amino acid, preferably Ala

<220>
<221>  MISC_FEATURE
<222>  (119)..(119)
<223>  Xaa can be any naturally occurring amino acid, preferably Thr

<220>
<221>  MISC_FEATURE
<222>  (125)..(125)
<223>  Xaa can be any naturally occurring amino acid, preferably Ser

<220>
<221>  MISC_FEATURE
<222>  (134)..(134)
<223>  Xaa can be any naturally occurring amino acid, preferably Ser

<220>
<221>  MISC_FEATURE
<222>  (142)..(142)
<223>  Xaa can be any naturally occurring amino acid, preferably Arg

<400>  16

Xaa Xaa Leu Ser Pro Ala Asp Lys Thr Asn Val Lys Ala Xaa Trp Gly
1               5                   10                  15

Xaa Val Gly Ala His Xaa Gly Glu Tyr Xaa Ala Glu Ala Leu Glu Xaa
            20                  25                  30

Met Phe Leu Ser Phe Xaa Xaa Thr Lys Thr Tyr Phe Pro His Phe Asp
        35                  40                  45

Leu Ser His Gly Ser Ala Xaa Val Lys Xaa His Gly Lys Lys Val Ala
    50                  55                  60

Xaa Xaa Leu Thr Asn Ala Val Xaa His Val Asp Xaa Xaa Pro Asn Ala
65                  70                  75                  80

Leu Ser Ala Leu Xaa Asp Leu His Ala His Lys Leu Xaa Val Asp Xaa
                85                  90                  95

Val Asn Phe Lys Leu Leu Ser His Cys Leu Xaa Xaa Thr Leu Xaa Ala
            100                 105                 110

His Leu Pro Ala Glu Phe Xaa Pro Ala Val His Ala Xaa Leu Asp Lys
        115                 120                 125

Phe Leu Ala Ser Val Xaa Thr Val Leu Thr Ser Lys Tyr Xaa
    130                 135                 140

<210>  17
<211>  117
<212>  PRT
<213>  Homo sapiens

<400>  17

Val Gly Ala His Ala Gly Glu Tyr Gly Ala Glu Ala Leu Glu Arg Met
1               5                   10                  15

Phe Leu Ser Phe Pro Thr Thr Lys Thr Tyr Phe Pro His Phe Asp Leu
            20                  25                  30

Ser His Gly Ser Ala Gln Val Lys Gly His Gly Lys Lys Val Ala Asp
        35                  40                  45

Ala Leu Thr Asn Ala Val Ala His Val Asp Asp Met Pro Asn Ala Leu
    50                  55                  60

Ser Ala Leu Ser Asp Leu His Ala His Lys Leu Arg Val Asp Pro Val
65                  70                  75                  80

Asn Phe Lys Leu Leu Ser His Cys Leu Leu Val Thr Leu Ala Ala His
                85                  90                  95

Leu Pro Ala Glu Phe Thr Pro Ala Val His Ala Ser Leu Asp Lys Phe
            100                 105                 110

Leu Ala Ser Val Ser
        115

<210>  18
<211>  142
<212>  PRT
<213>  Homo sapiens

<400>  18

<210>  19
<211>  124
<212>  PRT
<213>  Artificial Sequence

<220>
<223>  Minimal consensus sequence of mammalian haemoglobin beta chain

<220>
<221>  MISC_FEATURE
<222>  (4)..(4)
<223>  Xaa can be any naturally occurring amino acid, preferably Asn

<220>
<221>  MISC_FEATURE
<222>  (9)..(9)
<223>  Xaa can be any naturally occurring amino acid, preferably Gly

<220>
<221> MISC_FEATURE
<222> (10)..(10)
<223> Xaa can be any naturally occurring amino acid, preferably Gly

<220>
<221> MISC_FEATURE
<222> (12)..(12)
<223> Xaa can be absent or any naturally occurring amino acid,
      preferably Ala

<220>
<221> MISC_FEATURE
<222> (13)..(13)
<223> Xaa can be absent or any naturally occurring amino acid,
      preferably Leu

<220>
<221> MISC_FEATURE
<222> (14)..(14)
<223> Xaa can be absent or any naturally occurring amino acid,
      preferably Gly

<220>
<221> MISC_FEATURE
<222> (15)..(15)
<223> Xaa can be absent or any naturally occurring amino acid,
      preferably Arg

<220>
<221> MISC_FEATURE
<222> (27)..(27)
<223> Xaa can be absent or any naturally occurring amino acid,
      preferably Leu

<220>
<221> MISC_FEATURE
<222> (35)..(35)
<223> Xaa can be absent or any naturally occurring amino acid,
      preferably Thr

<220>
<221> MISC_FEATURE
<222> (36)..(36)
<223> Xaa can be absent or any naturally occurring amino acid,
      preferably Pro

<220>
<221> MISC_FEATURE
<222> (49)..(49)
<223> Xaa can be absent or any naturally occurring amino acid,
      preferably Gly

<220>
<221> MISC_FEATURE
<222> (52)..(52)
<223> Xaa can be absent or any naturally occurring amino acid,
      preferably Val

<220>
<221> MISC_FEATURE
<222> (66)..(66)
<223> Xaa can be absent or any naturally occurring amino acid,

preferably Leu

<220>
<221>  MISC_FEATURE
<222>  (68)..(68)
<223>  Xaa can be absent or any naturally occurring amino acid,
      preferably Gly

<220>
<221>  MISC_FEATURE
<222>  (69)..(69)
<223>  Xaa can be absent or any naturally occurring amino acid,
      preferably Thr

<220>
<221>  MISC_FEATURE
<222>  (86)..(86)
<223>  Xaa can be absent or any naturally occurring amino acid,
      preferably Glu

<220>
<221>  misc_feature
<222>  (121)..(121)
<223>  Xaa can be any naturally occurring amino acid

<220>
<221>  MISC_FEATURE
<222>  (122)..(122)
<223>  Xaa can be absent or any naturally occurring amino acid,
      preferably Val

<400>  19

Gly Lys Val Xaa Val Asp Glu Val Xaa Xaa Glu Xaa Xaa Xaa Xaa Leu
1               5                   10                  15

Leu Val Val Tyr Pro Trp Thr Gln Arg Phe Xaa Glu Ser Phe Gly Asp
            20                  25                  30

Leu Ser Xaa Xaa Asp Ala Val Met Gly Asn Pro Lys Val Lys Ala His
        35                  40                  45

Xaa Lys Lys Xaa Leu Gly Ala Phe Ser Asp Gly Leu Ala His Leu Asp
    50                  55                  60

Asn Xaa Lys Xaa Xaa Phe Ala Thr Leu Ser Glu Leu His Cys Asp Lys
65                  70                  75                  80

Leu His Val Asp Pro Xaa Asn Phe Arg Leu Leu Gly Asn Val Leu Val
                85                  90                  95

Cys Val Leu Ala His His Phe Gly Lys Glu Phe Thr Pro Pro Val Gln
            100                 105                 110

Ala Ala Tyr Gln Lys Val Val Ala Xaa Xaa Ala Asn
        115                 120

<210>  20
<211>  147
<212>  PRT
<213>  Artificial Sequence

<220>
<223>  Consensus sequence of mammalian haemoglobin beta chain



<220>
<221>  MISC_FEATURE
<222>  (10)..(10)
<223>  Xaa can be any naturally occurring amino acid, preferably Ser

<220>
<221>  MISC_FEATURE
<222>  (11)..(11)
<223>  Xaa can be any naturally occurring amino acid, preferably Ala

<220>
<221>  MISC_FEATURE
<222>  (15)..(15)
<223>  Xaa can be any naturally occurring amino acid, preferably Leu

<220>
<221>  MISC_FEATURE
<222>  (20)..(20)
<223>  Xaa can be any naturally occurring amino acid, preferably Asn

<220>
<221>  MISC_FEATURE
<222>  (25)..(25)
<223>  Xaa can be any naturally occurring amino acid, preferably Gly

<220>
<221>  MISC_FEATURE
<222>  (26)..(26)
<223>  Xaa can be any naturally occurring amino acid, preferably Gly

<220>
<221>  MISC_FEATURE
<222>  (28)..(28)
<223>  Xaa can be any naturally occurring amino acid, preferably Ala

<220>
<221>  MISC_FEATURE
<222>  (29)..(29)
<223>  Xaa can be any naturally occurring amino acid, preferably Leu

<220>
<221>  MISC_FEATURE
<222>  (30)..(30)
<223>  Xaa can be any naturally occurring amino acid, preferably Gly

<220>
<221>  MISC_FEATURE
<222>  (31)..(31)
<223>  Xaa can be any naturally occurring amino acid, preferably Arg

<220>
<221>  MISC_FEATURE

<222>  (43)..(43)
<223>  Xaa can be absent or any naturally occurring amino acid,
       preferably Leu

<220>
<221>  MISC_FEATURE
<222>  (51)..(51)
<223>  Xaa can be any naturally occurring amino acid, preferably Thr

<220>
<221>  MISC_FEATURE
<222>  (52)..(52)
<223>  Xaa can be any naturally occurring amino acid, preferably Pro

<220>
<221>  MISC_FEATURE
<222>  (65)..(65)
<223>  Xaa can be any naturally occurring amino acid, preferably Gly

<220>
<221>  MISC_FEATURE
<222>  (68)..(68)
<223>  Xaa can be any naturally occurring amino acid, preferably Val

<220>
<221>  MISC_FEATURE
<222>  (82)..(82)
<223>  Xaa can be any naturally occurring amino acid, preferably Leu

<220>
<221>  MISC_FEATURE
<222>  (84)..(84)
<223>  Xaa can be any naturally occurring amino acid, preferably Gly

<220>
<221>  MISC_FEATURE
<222>  (85)..(85)
<223>  Xaa can be any naturally occurring amino acid, preferably Thr

<220>
<221>  MISC_FEATURE
<222>  (102)..(102)
<223>  Xaa can be any naturally occurring amino acid, preferably Glu

<220>
<221>  MISC_FEATURE
<222>  (137)..(137)
<223>  Xaa can be any naturally occurring amino acid, preferably Gly

<220>
<221>  MISC_FEATURE
<222>  (138)..(138)
<223>  Xaa can be any naturally occurring amino acid, preferably Val

<220>
<221>  MISC_FEATURE
<222>  (147)..(147)
<223>  Xaa can be any naturally occurring amino acid, preferably His

<400>  20

Met Val His Leu Thr Pro Glu Glu Lys Xaa Xaa Val Thr Ala Xaa Trp
1               5                   10                  15

Gly Lys Val Xaa Val Asp Glu Val Xaa Xaa Glu Xaa Xaa Xaa Xaa Leu
            20                  25                  30

Leu Val Val Tyr Pro Trp Thr Gln Arg Phe Xaa Glu Ser Phe Gly Asp
        35                  40                  45

Leu Ser Xaa Xaa Asp Ala Val Met Gly Asn Pro Lys Val Lys Ala His
    50                  55                  60

Xaa Lys Lys Xaa Leu Gly Ala Phe Ser Asp Gly Leu Ala His Leu Asp
65                  70                  75                  80

Asn Xaa Lys Xaa Xaa Phe Ala Thr Leu Ser Glu Leu His Cys Asp Lys
                85                  90                  95

Leu His Val Asp Pro Xaa Asn Phe Arg Leu Leu Gly Asn Val Leu Val
            100                 105                 110

Cys Val Leu Ala His His Phe Gly Lys Glu Phe Thr Pro Pro Val Gln
        115                 120                 125

Ala Ala Tyr Gln Lys Val Val Ala Xaa Xaa Ala Asn Ala Leu Ala His
    130                 135                 140

Lys Tyr Xaa
145

<210> 21
<211> 124
<212> PRT
<213> Homo sapiens

<400> 21

Gly Lys Val Asn Val Asp Glu Val Gly Gly Glu Ala Leu Gly Arg Leu
1               5                   10                  15

Leu Val Val Tyr Pro Trp Thr Gln Arg Phe Leu Glu Ser Phe Gly Asp
            20                  25                  30

Leu Ser Thr Pro Asp Ala Val Met Gly Asn Pro Lys Val Lys Ala His
        35                  40                  45

Gly Lys Lys Val Leu Gly Ala Phe Ser Asp Gly Leu Ala His Leu Asp
    50                  55                  60

Asn Leu Lys Gly Thr Phe Ala Thr Leu Ser Glu Leu His Cys Asp Lys
65                  70                  75                  80

Leu His Val Asp Pro Glu Asn Phe Arg Leu Leu Gly Asn Val Leu Val
                85                  90                  95

Cys Val Leu Ala His His Phe Gly Lys Glu Phe Thr Pro Pro Val Gln
            100                 105                 110

Ala Ala Tyr Gln Lys Val Val Ala Gly Val Ala Asn
        115                 120

<210>  22
<211>  147
<212>  PRT
<213>  Homo sapiens

<400>  22

<210>  23
<211>  80

<212>  PRT
<213>  Artificial Sequence

<220>
<223>  N-terminal region of a consensus sequence of mammalian
      transferrins



<220>
<221>  MISC_FEATURE
<222>  (2)..(2)
<223>  Xaa can be any naturally occurring amino acid, preferably Arg

<220>
<221>  MISC_FEATURE
<222>  (9)..(9)
<223>  Xaa can be any naturally occurring amino acid, preferably Leu

<220>
<221>  MISC_FEATURE
<222>  (10)..(10)
<223>  Xaa can be any naturally occurring amino acid, preferably Val

<220>
<221>  MISC_FEATURE
<222>  (15)..(15)
<223>  Xaa can be any naturally occurring amino acid, preferably Gly

<220>
<221>  MISC_FEATURE
<222>  (24)..(24)
<223>  Xaa can be any naturally occurring amino acid, preferably Thr

<220>
<221>  MISC_FEATURE
<222>  (25)..(25)
<223>  Xaa can be any naturally occurring amino acid, preferably Val

<220>
<221>  MISC_FEATURE
<222>  (31)..(31)
<223>  Xaa can be any naturally occurring amino acid, preferably Ser

<220>
<221>  MISC_FEATURE
<222>  (32)..(32)
<223>  Xaa can be any naturally occurring amino acid, preferably Glu

<220>
<221>  MISC_FEATURE
<222>  (42)..(42)
<223>  Xaa can be any naturally occurring amino acid, preferably Arg

<220>
<221>  MISC_FEATURE
<222>  (47)..(47)
<223>  Xaa can be any naturally occurring amino acid, preferably Ser

<220>
<221>  misc_feature
<222>  (59)..(59)
<223>  Xaa can be any naturally occurring amino acid

<220>
<221>  MISC_FEATURE
<222>  (64)..(64)
<223>  Xaa can be any naturally occurring amino acid, preferably Tyr

<220>
<221>  MISC_FEATURE
<222>  (65)..(65)
<223>  Xaa can be any naturally occurring amino acid, preferably Leu

<220>
<221>  MISC_FEATURE
<222>  (71)..(71)
<223>  Xaa can be any naturally occurring amino acid, preferably Ile

<220>
<221>  MISC_FEATURE
<222>  (73)..(73)
<223>  Xaa can be any naturally occurring amino acid, preferably Ala

<220>
<221>  MISC_FEATURE
<222>  (77)..(77)
<223>  Xaa can be any naturally occurring amino acid, preferably Asp

<220>
<221>  MISC_FEATURE
<222>  (78)..(78)
<223>  Xaa can be any naturally occurring amino acid, preferably Ala

<400>  23

Met Xaa Leu Ala Val Gly Ala Leu Xaa Xaa Cys Ala Val Leu Xaa Leu
1               5                   10                  15

Cys Leu Ala Val Pro Asp Lys Xaa Xaa Arg Trp Cys Ala Val Xaa Xaa
            20                  25                  30

His Glu Ala Thr Lys Cys Gln Ser Phe Xaa Asp His Met Lys Xaa Val
        35                  40                  45

Ile Pro Ser Asp Gly Pro Ser Val Ala Cys Xaa Lys Lys Ala Ser Xaa
    50                  55                  60

Xaa Asp Cys Ile Arg Ala Xaa Ala Xaa Asn Glu Ala Xaa Xaa Val Thr
65                  70                  75                  80

<210>  24
<211>  114
<212>  PRT
<213>  Artificial Sequence

<220>
<223>  C-terminal region of a consensus sequence of mammalian
       transferrins

<220>

<221>  MISC_FEATURE
<222>  (3)..(3)
<223>  Xaa can be any naturally occurring amino acid, preferably Thr

<220>
<221>  MISC_FEATURE
<222>  (16)..(16)
<223>  Xaa can be any naturally occurring amino acid, preferably Asp

<220>
<221>  MISC_FEATURE
<222>  (18)..(18)
<223>  Xaa can be any naturally occurring amino acid, preferably Met

<220>
<221>  MISC_FEATURE
<222>  (19)..(19)
<223>  Xaa can be any naturally occurring amino acid, preferably Ser

<220>
<221>  MISC_FEATURE
<222>  (23)..(23)
<223>  Xaa can be any naturally occurring amino acid, preferably Gly

<220>
<221>  MISC_FEATURE
<222>  (25)..(25)
<223>  Xaa can be any naturally occurring amino acid, preferably Val

<220>
<221>  MISC_FEATURE
<222>  (28)..(28)
<223>  Xaa can be any naturally occurring amino acid, preferably Ala

<220>
<221>  MISC_FEATURE
<222>  (30)..(30)
<223>  Xaa can be any naturally occurring amino acid, preferably Lys

<220>
<221>  MISC_FEATURE
<222>  (61)..(61)
<223>  Xaa can be any naturally occurring amino acid, preferably Val

<220>
<221>  MISC_FEATURE
<222>  (62)..(62)
<223>  Xaa can be any naturally occurring amino acid, preferably Lys

<220>
<221>  MISC_FEATURE
<222>  (75)..(75)
<223>  Xaa can be any naturally occurring amino acid, preferably Gly

<220>
<221>  MISC_FEATURE
<222>  (78)..(78)
<223>  Xaa can be any naturally occurring amino acid, preferably Ser

<220>
<221>  MISC_FEATURE
<222>  (96)..(96)
<223>  Xaa can be any naturally occurring amino acid, preferably Leu

<220>
<221>  MISC_FEATURE
<222>  (97)..(97)
<223>  Xaa can be any naturally occurring amino acid, preferably Tyr

<220>
<221>  MISC_FEATURE
<222>  (105)..(105)
<223>  Xaa can be any naturally occurring amino acid, preferably Phe

<220>
<221>  MISC_FEATURE
<222>  (107)..(107)
<223>  Xaa can be any naturally occurring amino acid, preferably Glu

<220>
<221>  MISC_FEATURE
<222>  (110)..(110)
<223>  Xaa can be any naturally occurring amino acid, preferably Ser

<400>  24

Glu Thr Xaa Glu Asp Cys Ile Ala Lys Ile Met Asn Gly Glu Ala Xaa
1               5                   10                  15

Ala Xaa Xaa Leu Asp Gly Xaa Phe Xaa Tyr Ile Xaa Gly Xaa Cys Gly
            20                  25                  30

Leu Val Pro Val Leu Ala Glu Asn Tyr Asn Lys Ser Asp Asn Cys Glu
        35                  40                  45

Asp Thr Pro Glu Ala Gly Tyr Phe Ala Val Ala Val Xaa Xaa Lys Ser
    50                  55                  60

Ala Ser Asp Leu Thr Trp Asp Asn Leu Lys Xaa Lys Lys Xaa Cys His
65                  70                  75                  80

Thr Ala Val Gly Arg Thr Ala Gly Trp Asn Ile Pro Met Gly Leu Xaa
                85                  90                  95

Xaa Asn Lys Ile Asn His Cys Arg Xaa Asp Xaa Phe Phe Xaa Glu Gly
            100                 105                 110

Cys Ala

<210>  25
<211>  698
<212>  PRT
<213>  Artificial Sequence

<220>
<223>  Consensus sequence of mammalian transferrins

<220>
<221>  MISC_FEATURE
<222>  (2)..(2)
<223>  Xaa can be any naturally occurring amino acid, preferably Arg

<220>
<221>  misc_feature
<222>  (9)..(9)
<223>  Xaa can be any naturally occurring amino acid

<220>
<221>  MISC_FEATURE
<222>  (10)..(10)
<223>  Xaa can be any naturally occurring amino acid, preferably Val

<220>
<221>  MISC_FEATURE
<222>  (15)..(15)
<223>  Xaa can be any naturally occurring amino acid, preferably Gly

<220>
<221>  MISC_FEATURE
<222>  (24)..(24)
<223>  Xaa can be any naturally occurring amino acid, preferably Thr

<220>
<221>  MISC_FEATURE
<222>  (25)..(25)
<223>  Xaa can be any naturally occurring amino acid, preferably Val

<220>
<221>  MISC_FEATURE
<222>  (31)..(31)
<223>  Xaa can be any naturally occurring amino acid, preferably Ser

<220>
<221>  MISC_FEATURE
<222>  (32)..(32)
<223>  Xaa can be any naturally occurring amino acid, preferably Glu

<220>
<221>  MISC_FEATURE
<222>  (42)..(42)
<223>  Xaa can be any naturally occurring amino acid, preferably Arg

<220>
<221>  MISC_FEATURE
<222>  (47)..(47)
<223>  Xaa can be any naturally occurring amino acid, preferably Ser

<220>
<221>  MISC_FEATURE
<222>  (59)..(59)
<223>  Xaa can be any naturally occurring amino acid, preferably Val

<220>
<221>  MISC_FEATURE
<222>  (64)..(64)
<223>  Xaa can be any naturally occurring amino acid, preferably Tyr

<220>
<221>  MISC_FEATURE

<222>  (65)..(65)
<223>  Xaa can be any naturally occurring amino acid, preferably Leu

<220>
<221>  misc_feature
<222>  (71)..(71)
<223>  Xaa can be any naturally occurring amino acid

<220>
<221>  MISC_FEATURE
<222>  (73)..(73)
<223>  Xaa can be any naturally occurring amino acid, preferably Ile

<220>
<221>  MISC_FEATURE
<222>  (77)..(77)
<223>  Xaa can be any naturally occurring amino acid, preferably Asp

<220>
<221>  MISC_FEATURE
<222>  (78)..(78)
<223>  Xaa can be any naturally occurring amino acid, preferably Ala

<220>
<221>  MISC_FEATURE
<222>  (84)..(84)
<223>  Xaa can be any naturally occurring amino acid, preferably Gly

<220>
<221>  MISC_FEATURE
<222>  (87)..(87)
<223>  Xaa can be any naturally occurring amino acid, preferably Tyr

<220>
<221>  MISC_FEATURE
<222>  (88)..(88)
<223>  Xaa can be any naturally occurring amino acid, preferably Asp

<220>
<221>  MISC_FEATURE
<222>  (94)..(94)
<223>  Xaa can be any naturally occurring amino acid, preferably Asn

<220>
<221>  MISC_FEATURE
<222>  (98)..(98)
<223>  Xaa can be any naturally occurring amino acid, preferably Pro

<220>
<221>  MISC_FEATURE
<222>  (104)..(104)
<223>  Xaa can be any naturally occurring amino acid, preferably Tyr

<220>
<221>  MISC_FEATURE
<222>  (107)..(107)
<223>  Xaa can be any naturally occurring amino acid, preferably Lys

<220>
<221>  MISC_FEATURE
<222>  (108)..(108)
<223>  Xaa can be any naturally occurring amino acid, preferably Glu

<220>
<221>  MISC_FEATURE
<222>  (115)..(115)
<223>  Xaa can be any naturally occurring amino acid, preferably Tyr

<220>
<221>  MISC_FEATURE
<222>  (116)..(116)
<223>  Xaa can be any naturally occurring amino acid, preferably Ala

<220>
<221>  MISC_FEATURE
<222>  (121)..(121)
<223>  Xaa can be any naturally occurring amino acid, preferably Lys

<220>
<221>  MISC_FEATURE
<222>  (124)..(124)
<223>  Xaa can be any naturally occurring amino acid, preferably Ser

<220>
<221>  MISC_FEATURE
<222>  (129)..(129)
<223>  Xaa can be any naturally occurring amino acid, preferably Asn

<220>
<221>  MISC_FEATURE
<222>  (146)..(146)
<223>  Xaa can be any naturally occurring amino acid, preferably Gly

<220>
<221>  MISC_FEATURE
<222>  (152)..(152)
<223>  Xaa can be any naturally occurring amino acid, preferably Gly

<220>
<221>  MISC_FEATURE
<222>  (153)..(153)
<223>  Xaa can be any naturally occurring amino acid, preferably Leu

<220>
<221>  MISC_FEATURE
<222>  (161)..(161)
<223>  Xaa can be any naturally occurring amino acid, preferably Pro

<220>
<221>  MISC_FEATURE
<222>  (179)..(179)
<223>  Xaa can be any naturally occurring amino acid, preferably Pro

<220>
<221>  MISC_FEATURE
<222>  (188)..(188)
<223>  Xaa can be any naturally occurring amino acid, preferably Gln

<220>
<221>  MISC_FEATURE
<222>  (204)..(204)
<223>  Xaa can be any naturally occurring amino acid, preferably Tyr

<220>
<221>  MISC_FEATURE
<222>  (224)..(224)

<223>  Xaa can be any naturally occurring amino acid, preferably Val

<220>
<221>  MISC_FEATURE
<222>  (238)..(238)
<223>  Xaa can be any naturally occurring amino acid, preferably Asp

<220>
<221>  MISC_FEATURE
<222>  (245)..(245)
<223>  Xaa can be any naturally occurring amino acid, preferably Leu

<220>
<221>  MISC_FEATURE
<222>  (255)..(255)
<223>  Xaa can be any naturally occurring amino acid, preferably Asp

<220>
<221>  MISC_FEATURE
<222>  (268)..(268)
<223>  Xaa can be any naturally occurring amino acid, preferably His

<220>
<221>  MISC_FEATURE
<222>  (275)..(275)
<223>  Xaa can be any naturally occurring amino acid, preferably Met

<220>
<221>  MISC_FEATURE
<222>  (276)..(276)
<223>  Xaa can be any naturally occurring amino acid, preferably Gly

<220>
<221>  MISC_FEATURE
<222>  (286)..(286)
<223>  Xaa can be any naturally occurring amino acid, preferably Leu

<220>
<221>  MISC_FEATURE
<222>  (293)..(293)
<223>  Xaa can be any naturally occurring amino acid, preferably Phe

<220>
<221>  MISC_FEATURE
<222>  (309)..(309)
<223>  Xaa can be any naturally occurring amino acid, preferably Gly

<220>
<221>  MISC_FEATURE
<222>  (310)..(310)
<223>  Xaa can be any naturally occurring amino acid, preferably Lys

<220>
<221>  MISC_FEATURE
<222>  (326)..(326)
<223>  Xaa can be any naturally occurring amino acid, preferably Pro

<220>
<221>  MISC_FEATURE
<222>  (327)..(327)
<223>  Xaa can be any naturally occurring amino acid, preferably Arg

<220>

<221>  MISC_FEATURE
<222>  (357)..(357)
<223>  Xaa can be any naturally occurring amino acid, preferably Glu

<220>
<221>  MISC_FEATURE
<222>  (381)..(381)
<223>  Xaa can be any naturally occurring amino acid, preferably Ser

<220>
<221>  MISC_FEATURE
<222>  (393)..(393)
<223>  Xaa can be any naturally occurring amino acid, preferably Thr

<220>
<221>  MISC_FEATURE
<222>  (406)..(406)
<223>  Xaa can be any naturally occurring amino acid, preferably Asp

<220>
<221>  MISC_FEATURE
<222>  (408)..(408)
<223>  Xaa can be any naturally occurring amino acid, preferably Met

<220>
<221>  MISC_FEATURE
<222>  (409)..(409)
<223>  Xaa can be any naturally occurring amino acid, preferably Ser

<220>
<221>  MISC_FEATURE
<222>  (413)..(413)
<223>  Xaa can be any naturally occurring amino acid, preferably Gly

<220>
<221>  MISC_FEATURE
<222>  (415)..(415)
<223>  Xaa can be any naturally occurring amino acid, preferably Val

<220>
<221>  MISC_FEATURE
<222>  (418)..(418)
<223>  Xaa can be any naturally occurring amino acid, preferably Ala

<220>
<221>  MISC_FEATURE
<222>  (420)..(420)
<223>  Xaa can be any naturally occurring amino acid, preferably Lys

<220>
<221>  MISC_FEATURE
<222>  (451)..(451)
<223>  Xaa can be any naturally occurring amino acid, preferably Val

<220>
<221>  MISC_FEATURE
<222>  (452)..(452)
<223>  Xaa can be any naturally occurring amino acid, preferably Lys

<220>
<221>  MISC_FEATURE
<222>  (465)..(465)
<223>  Xaa can be any naturally occurring amino acid, preferably Gly

<220>
<221>  MISC_FEATURE
<222>  (468)..(468)
<223>  Xaa can be any naturally occurring amino acid, preferably Ser

<220>
<221>  MISC_FEATURE
<222>  (486)..(486)
<223>  Xaa can be any naturally occurring amino acid, preferably Leu

<220>
<221>  MISC_FEATURE
<222>  (487)..(487)
<223>  Xaa can be any naturally occurring amino acid, preferably Tyr

<220>
<221>  MISC_FEATURE
<222>  (495)..(495)
<223>  Xaa can be any naturally occurring amino acid, preferably Phe

<220>
<221>  MISC_FEATURE
<222>  (497)..(497)
<223>  Xaa can be any naturally occurring amino acid, preferably Glu

<220>
<221>  MISC_FEATURE
<222>  (500)..(500)
<223>  Xaa can be any naturally occurring amino acid, preferably Ser

<220>
<221>  MISC_FEATURE
<222>  (505)..(505)
<223>  Xaa can be any naturally occurring amino acid, preferably Pro

<220>
<221>  MISC_FEATURE
<222>  (509)..(509)
<223>  Xaa can be any naturally occurring amino acid, preferably Lys

<220>
<221>  MISC_FEATURE
<222>  (529)..(529)
<223>  Xaa can be any naturally occurring amino acid, preferably Asn

<220>
<221>  MISC_FEATURE
<222>  (536)..(536)
<223>  Xaa can be any naturally occurring amino acid, preferably Tyr

<220>
<221>  MISC_FEATURE
<222>  (558)..(558)
<223>  Xaa can be any naturally occurring amino acid, preferably Pro

<220>
<221>  misc_feature
<222>  (559)..(559)
<223>  Xaa can be any naturally occurring amino acid

<220>
<221>  MISC_FEATURE

<222>  (563)..(563)
<223>  Xaa can be any naturally occurring amino acid, preferably Gly

<220>
<221>  MISC_FEATURE
<222>  (586)..(586)
<223>  Xaa can be any naturally occurring amino acid, preferably Thr

<220>
<221>  MISC_FEATURE
<222>  (592)..(592)
<223>  Xaa can be any naturally occurring amino acid, preferably Glu

<220>
<221>  MISC_FEATURE
<222>  (603)..(603)
<223>  Xaa can be any naturally occurring amino acid, preferably Asn

<220>
<221>  MISC_FEATURE
<222>  (607)..(607)
<223>  Xaa can be any naturally occurring amino acid, preferably Val

<220>
<221>  MISC_FEATURE
<222>  (622)..(622)
<223>  Xaa can be any naturally occurring amino acid, preferably Gln

<220>
<221>  MISC_FEATURE
<222>  (635)..(635)
<223>  Xaa can be any naturally occurring amino acid, preferably Ser

<220>
<221>  MISC_FEATURE
<222>  (648)..(648)
<223>  Xaa can be any naturally occurring amino acid, preferably Leu

<220>
<221>  MISC_FEATURE
<222>  (652)..(652)
<223>  Xaa can be any naturally occurring amino acid, preferably Asp

<220>
<221>  MISC_FEATURE
<222>  (678)..(678)
<223>  Xaa can be any naturally occurring amino acid, preferably Val

<220>
<221>  MISC_FEATURE
<222>  (692)..(692)
<223>  Xaa can be any naturally occurring amino acid, preferably Ala

<220>
<221>  MISC_FEATURE
<222>  (696)..(696)
<223>  Xaa can be any naturally occurring amino acid, preferably Arg

<220>
<221>  MISC_FEATURE
<222>  (698)..(698)
<223>  Xaa can be any naturally occurring amino acid, preferably Pro

<400> 25

Met Xaa Leu Ala Val Gly Ala Leu Xaa Xaa Cys Ala Val Leu Xaa Leu
1               5                   10                  15

Cys Leu Ala Val Pro Asp Lys Xaa Xaa Arg Trp Cys Ala Val Xaa Xaa
            20                  25                  30

His Glu Ala Thr Lys Cys Gln Ser Phe Xaa Asp His Met Lys Xaa Val
        35                  40                  45

Ile Pro Ser Asp Gly Pro Ser Val Ala Cys Xaa Lys Lys Ala Ser Xaa
    50                  55                  60

Xaa Asp Cys Ile Arg Ala Xaa Ala Xaa Asn Glu Ala Xaa Xaa Val Thr
65                  70                  75                  80

Leu Asp Ala Xaa Leu Val Xaa Xaa Ala Tyr Leu Ala Pro Xaa Asn Leu
                85                  90                  95

Lys Xaa Val Val Ala Glu Phe Xaa Gly Ser Xaa Xaa Asp Pro Gln Thr
            100                 105                 110

Phe Tyr Xaa Xaa Val Ala Val Val Xaa Lys Asp Xaa Gly Phe Gln Met
        115                 120                 125

Xaa Gln Leu Arg Gly Lys Lys Ser Cys His Thr Gly Leu Gly Arg Ser
    130                 135                 140

Ala Xaa Trp Asn Ile Pro Ile Xaa Xaa Leu Tyr Cys Asp Leu Pro Glu
145                 150                 155                 160

Xaa Arg Lys Pro Leu Glu Lys Ala Val Ala Asn Phe Phe Ser Gly Ser
                165                 170                 175

Cys Ala Xaa Cys Ala Asp Gly Thr Asp Phe Pro Xaa Leu Cys Gln Leu
            180                 185                 190

Cys Pro Gly Cys Gly Cys Ser Thr Leu Asn Gln Xaa Phe Gly Tyr Ser
        195                 200                 205

Gly Ala Phe Lys Cys Leu Lys Asp Gly Ala Gly Asp Val Ala Phe Xaa
    210                 215                 220

Lys His Ser Thr Ile Phe Glu Asn Leu Ala Asn Lys Ala Xaa Arg Asp
225                 230                 235                 240

Gln Tyr Glu Leu Xaa Cys Leu Asp Asn Thr Arg Lys Pro Val Xaa Glu

                245                 250                 255

Tyr Lys Asp Cys His Leu Ala Gln Val Pro Ser Xaa Thr Val Val Ala
            260                 265                 270

Arg Ser Xaa Xaa Gly Lys Glu Asp Leu Ile Trp Glu Leu Xaa Asn Gln
        275                 280                 285

Ala Gln Glu His Xaa Gly Lys Asp Lys Ser Lys Glu Phe Gln Leu Phe
    290                 295                 300

Ser Ser Pro His Xaa Xaa Asp Leu Leu Phe Lys Asp Ser Ala His Gly
305                 310                 315                 320

Phe Leu Lys Val Pro Xaa Xaa Met Asp Ala Lys Met Tyr Leu Gly Tyr
                325                 330                 335

Glu Tyr Val Thr Ala Ile Arg Asn Leu Arg Glu Gly Thr Cys Pro Glu
            340                 345                 350

Ala Pro Thr Asp Xaa Cys Lys Pro Val Lys Trp Cys Ala Leu Ser His
        355                 360                 365

His Glu Arg Leu Lys Cys Asp Glu Trp Ser Val Asn Xaa Val Gly Lys
    370                 375                 380

Ile Glu Cys Val Ser Ala Glu Thr Xaa Glu Asp Cys Ile Ala Lys Ile
385                 390                 395                 400

Met Asn Gly Glu Ala Xaa Ala Xaa Xaa Leu Asp Gly Xaa Phe Xaa Tyr
                405                 410                 415

Ile Xaa Gly Xaa Cys Gly Leu Val Pro Val Leu Ala Glu Asn Tyr Asn
            420                 425                 430

Lys Ser Asp Asn Cys Glu Asp Thr Pro Glu Ala Gly Tyr Phe Ala Val
        435                 440                 445

Ala Val Xaa Xaa Lys Ser Ala Ser Asp Leu Thr Trp Asp Asn Leu Lys
    450                 455                 460

Xaa Lys Lys Xaa Cys His Thr Ala Val Gly Arg Thr Ala Gly Trp Asn
465                 470                 475                 480

Ile Pro Met Gly Leu Xaa Xaa Asn Lys Ile Asn His Cys Arg Xaa Asp
                485                 490                 495

Xaa Phe Phe Xaa Glu Gly Cys Ala Xaa Gly Ser Lys Xaa Asp Ser Ser
        500                 505                 510

Leu Cys Lys Leu Cys Met Gly Ser Gly Leu Asn Leu Cys Glu Pro Asn
    515                 520                 525

Xaa Lys Glu Gly Tyr Tyr Gly Xaa Thr Gly Ala Phe Arg Cys Leu Val
    530                 535                 540

Glu Lys Gly Asp Val Ala Phe Val Lys His Gln Thr Val Xaa Xaa Asn
545                 550                 555                 560

Thr Gly Xaa Lys Asn Pro Asp Pro Trp Ala Lys Asn Leu Asn Glu Lys
                565                 570                 575

Asp Tyr Glu Leu Leu Cys Leu Asp Gly Xaa Arg Lys Pro Val Glu Xaa
            580                 585                 590

Tyr Ala Asn Cys His Leu Ala Arg Ala Pro Xaa His Ala Val Xaa Thr
        595                 600                 605

Arg Lys Asp Lys Glu Ala Cys Val His Lys Ile Leu Arg Xaa Gln Gln
    610                 615                 620

His Leu Phe Gly Ser Asn Val Thr Asp Cys Xaa Gly Asn Phe Cys Leu
625                 630                 635                 640

Phe Arg Ser Glu Thr Lys Asp Xaa Leu Phe Arg Xaa Asp Thr Val Cys
                645                 650                 655

Leu Ala Lys Leu His Asp Arg Asn Thr Tyr Glu Lys Tyr Leu Gly Glu
            660                 665                 670

Glu Tyr Val Lys Ala Xaa Gly Asn Leu Arg Lys Cys Ser Thr Ser Ser
        675                 680                 685

Leu Leu Glu Xaa Cys Thr Phe Xaa Arg Xaa
    690                 695

<210>  26
<211>  80
<212>  PRT
<213>  Homo sapiens

<400>  26

Met Arg Leu Ala Val Gly Ala Leu Leu Val Cys Ala Val Leu Gly Leu
1               5                   10                  15

Cys Leu Ala Val Pro Asp Lys Thr Val Arg Trp Cys Ala Val Ser Glu
            20                  25                  30

His Glu Ala Thr Lys Cys Gln Ser Phe Arg Asp His Met Lys Ser Val
        35                  40                  45

Ile Pro Ser Asp Gly Pro Ser Val Ala Cys Val Lys Lys Ala Ser Tyr
    50                  55                  60

Leu Asp Cys Ile Arg Ala Ile Ala Ala Asn Glu Ala Asp Ala Val Thr
65                  70                  75                  80

<210>  27
<211>  114
<212>  PRT
<213>  Homo sapiens

<400>  27

Glu Thr Thr Glu Asp Cys Ile Ala Lys Ile Met Asn Gly Glu Ala Asp
1               5                   10                  15

Ala Met Ser Leu Asp Gly Gly Phe Val Tyr Ile Ala Gly Lys Cys Gly
            20                  25                  30

Leu Val Pro Val Leu Ala Glu Asn Tyr Asn Lys Ser Asp Asn Cys Glu
        35                  40                  45

Asp Thr Pro Glu Ala Gly Tyr Phe Ala Val Ala Val Val Lys Lys Ser
    50                  55                  60

Ala Ser Asp Leu Thr Trp Asp Asn Leu Lys Gly Lys Lys Ser Cys His
65                  70                  75                  80

Thr Ala Val Gly Arg Thr Ala Gly Trp Asn Ile Pro Met Gly Leu Leu
                85                  90                  95

Tyr Asn Lys Ile Asn His Cys Arg Phe Asp Glu Phe Phe Ser Glu Gly
            100                 105                 110

Cys Ala

<210>  28
<211>  698
<212>  PRT
<213>  Homo sapiens

<400>  28

Met Arg Leu Ala Val Gly Ala Leu Leu Val Cys Ala Val Leu Gly Leu

1               5                   10                  15

Cys Leu Ala Val Pro Asp Lys Thr Val Arg Trp Cys Ala Val Ser Glu
            20                  25                  30

His Glu Ala Thr Lys Cys Gln Ser Phe Arg Asp His Met Lys Ser Val
        35                  40                  45

Ile Pro Ser Asp Gly Pro Ser Val Ala Cys Val Lys Lys Ala Ser Tyr
    50                  55                  60

Leu Asp Cys Ile Arg Ala Ile Ala Ala Asn Glu Ala Asp Ala Val Thr
65                  70                  75                  80

Leu Asp Ala Gly Leu Val Tyr Asp Ala Tyr Leu Ala Pro Asn Asn Leu
                85                  90                  95

Lys Pro Val Val Ala Glu Phe Tyr Gly Ser Lys Glu Asp Pro Gln Thr
            100                 105                 110

Phe Tyr Tyr Ala Val Ala Val Val Lys Lys Asp Ser Gly Phe Gln Met
        115                 120                 125

Asn Gln Leu Arg Gly Lys Lys Ser Cys His Thr Gly Leu Gly Arg Ser
    130                 135                 140

Ala Gly Trp Asn Ile Pro Ile Gly Leu Leu Tyr Cys Asp Leu Pro Glu
145                 150                 155                 160

Pro Arg Lys Pro Leu Glu Lys Ala Val Ala Asn Phe Phe Ser Gly Ser
                165                 170                 175

Cys Ala Pro Cys Ala Asp Gly Thr Asp Phe Pro Gln Leu Cys Gln Leu
            180                 185                 190

Cys Pro Gly Cys Gly Cys Ser Thr Leu Asn Gln Tyr Phe Gly Tyr Ser
        195                 200                 205

Gly Ala Phe Lys Cys Leu Lys Asp Gly Ala Gly Asp Val Ala Phe Val
    210                 215                 220

Lys His Ser Thr Ile Phe Glu Asn Leu Ala Asn Lys Ala Asp Arg Asp
225                 230                 235                 240

Gln Tyr Glu Leu Leu Cys Leu Asp Asn Thr Arg Lys Pro Val Asp Glu
                245                 250                 255

Tyr Lys Asp Cys His Leu Ala Gln Val Pro Ser His Thr Val Val Ala
            260                 265                 270

Arg Ser Met Gly Gly Lys Glu Asp Leu Ile Trp Glu Leu Leu Asn Gln
        275                 280                 285

Ala Gln Glu His Phe Gly Lys Asp Lys Ser Lys Glu Phe Gln Leu Phe
    290                 295                 300

Ser Ser Pro His Gly Lys Asp Leu Leu Phe Lys Asp Ser Ala His Gly
305                 310                 315                 320

Phe Leu Lys Val Pro Pro Arg Met Asp Ala Lys Met Tyr Leu Gly Tyr
                325                 330                 335

Glu Tyr Val Thr Ala Ile Arg Asn Leu Arg Glu Gly Thr Cys Pro Glu
            340                 345                 350

Ala Pro Thr Asp Glu Cys Lys Pro Val Lys Trp Cys Ala Leu Ser His
        355                 360                 365

His Glu Arg Leu Lys Cys Asp Glu Trp Ser Val Asn Ser Val Gly Lys
    370                 375                 380

Ile Glu Cys Val Ser Ala Glu Thr Thr Glu Asp Cys Ile Ala Lys Ile
385                 390                 395                 400

Met Asn Gly Glu Ala Asp Ala Met Ser Leu Asp Gly Gly Phe Val Tyr
                405                 410                 415

Ile Ala Gly Lys Cys Gly Leu Val Pro Val Leu Ala Glu Asn Tyr Asn
            420                 425                 430

Lys Ser Asp Asn Cys Glu Asp Thr Pro Glu Ala Gly Tyr Phe Ala Val
        435                 440                 445

Ala Val Val Lys Lys Ser Ala Ser Asp Leu Thr Trp Asp Asn Leu Lys
    450                 455                 460

Gly Lys Lys Ser Cys His Thr Ala Val Gly Arg Thr Ala Gly Trp Asn
465                 470                 475                 480

Ile Pro Met Gly Leu Leu Tyr Asn Lys Ile Asn His Cys Arg Phe Asp
                485                 490                 495

Glu Phe Phe Ser Glu Gly Cys Ala Pro Gly Ser Lys Lys Asp Ser Ser
            500                 505                 510

Leu Cys Lys Leu Cys Met Gly Ser Gly Leu Asn Leu Cys Glu Pro Asn
        515                 520                 525

Asn Lys Glu Gly Tyr Tyr Gly Tyr Thr Gly Ala Phe Arg Cys Leu Val
    530                 535                 540

Glu Lys Gly Asp Val Ala Phe Val Lys His Gln Thr Val Pro Gln Asn
545                 550                 555                 560

Thr Gly Gly Lys Asn Pro Asp Pro Trp Ala Lys Asn Leu Asn Glu Lys
                565                 570                 575

Asp Tyr Glu Leu Leu Cys Leu Asp Gly Thr Arg Lys Pro Val Glu Glu
            580                 585                 590

Tyr Ala Asn Cys His Leu Ala Arg Ala Pro Asn His Ala Val Val Thr
        595                 600                 605

Arg Lys Asp Lys Glu Ala Cys Val His Lys Ile Leu Arg Gln Gln Gln
    610                 615                 620

His Leu Phe Gly Ser Asn Val Thr Asp Cys Ser Gly Asn Phe Cys Leu
625                 630                 635                 640

Phe Arg Ser Glu Thr Lys Asp Leu Leu Phe Arg Asp Asp Thr Val Cys
                645                 650                 655

Leu Ala Lys Leu His Asp Arg Asn Thr Tyr Glu Lys Tyr Leu Gly Glu
            660                 665                 670

Glu Tyr Val Lys Ala Val Gly Asn Leu Arg Lys Cys Ser Thr Ser Ser
        675                 680                 685

Leu Leu Glu Ala Cys Thr Phe Arg Arg Pro
    690                 695